# FASCICULE DE BREVET EUROPEEN

(11) **EP 3 558 381 B1**
(45) Date de publication et mention de la délivrance du brevet: **07.04.2021**
(21) Numéro de dépôt: 17837979.8
(22) Date de dépôt: 22.12.2017
(51) Int. Cl.: A61K 38/36, A61K 31/198, A61P 41/00, A61P 7/04, A61K 9/00, A61K 47/18, A61K 47/26

(54) **COMPOSITION LIQUIDE DE FIBRINOGENE HUMAIN**
FLÜSSIGE HUMANE FIBRINOGENZUSAMMENSETZUNG
LIQUID HUMAN FIBRINOGEN COMPOSITION

(30) Priorité: 22.12.2016 FR 1663181
(43) Date de publication de la demande: 30.10.2019
(73) Titulaire: Laboratoire Français du Fractionnement et des Biotechnologies, 91940 Les Ulis (FR)
(72) Inventeur: DEBRAIZE, Clémence, 91940 Les Ulis (FR)
(74) Mandataire: Plasseraud IP
(86) Numéro de dépôt international: PCT/FR2017/053839
(87) Numéro de publication internationale: WO 2018/115800

(56) Documents cités:
- EP-A1- 2 130 549
- FR-A1- 2 857 267
- US-A1- 2005 080 009
- MAO S J T ET AL: "CHARACTERIZATION OF A MONOCLONAL ANTIBODY SPECIFIC TO THE AMINO TERMINUS OF THE ALPHA-CHAIN OF HUMAN FIBRIN", THROMBOSIS AND HAEMOSTASIS, SCHATTAUER GMBH, DE, vol. 63, no. 3, 28 mars 1990 (1990-03-28), pages 445-448, XP000979448, ISSN: 0340-6245
- KHEDDO PRISCILLA ET AL: "The effect of arginine glutamate on the stability of monoclonal antibodies in solution.", INTERNATIONAL JOURNAL OF PHARMACEUTICS 01 OCT 2014, vol. 473, no. 1-2, 1 octobre 2014 (2014-10-01), pages 126-133, XP002771064, ISSN: 1873-3476

## Description

L'invention a trait à une formulation de fibrinogène humain, utile en thérapie.

De nombreuses pathologies sont actuellement traitées par des compositions comprenant du fibrinogène. On peut citer par exemple les hypo-fibrinogénémies, dys-fibrinogénémies ou a-fibrinogénémies constitutionnelles chez les patients présentant une hémorragie spontanée ou post-traumatique, le traitement complémentaire dans la prise en charge d'une hémorragie sévère incontrôlée dans le cadre d'une hypofibrinogénémie acquise, etc.

Pour le traitement de certaines pathologies, l'utilisation de compositions comprenant du fibrinogène stables au stockage et prêtes à l'emploi peut s'avérer particulièrement avantageuse. Cette forme d'administration offre en effet plus de flexibilité et de rapidité d'administration aux praticiens, améliorant la prise en charge urgente des patients hémorragiques. A cette fin, des compositions comprenant du fibrinogène lyophilisées, stables au stockage et adaptées à une constitution rapide ont été développées. Cependant, la reconstitution de telles compositions lyophilisées nécessite quelques minutes. De plus, la reconstitution doit être réalisée doucement pour permettre la dissolution complète du lyophilisat, garantissant la concentration du produit, et ce sans formation de mousse, de trouble ou de dépôt qui rendrait la composition difficilement ou non administrable. L'utilisation de tels produits lyophilisés n'est donc pas optimale dans un contexte de médecine d'urgence intra ou péri-hospitalière où chaque minute compte pour le traitement de l'hémorragie.

A ce jour, les compositions comprenant du fibrinogène ne donnent pas entière satisfaction en termes de stabilité liquide notamment.

Dans ce contexte, les besoins en compositions liquides comprenant du fibrinogène d'utilisation aisée, persistent.

### Résumé de l'invention :

En travaillant sur les problèmes de stabilité propres aux compositions comprenant du fibrinogène, la Demanderesse a mis au point une formulation particulière, combinant du fibrinogène, de l'arginine et du glutamate, dans des ratios particuliers participant à la stabilité sous forme liquide de ladite formulation dans le temps. De manière surprenante et avantageuse, la Demanderesse a mis en évidence qu'il est possible d'obtenir des compositions comprenant du fibrinogène particulièrement stables dans le temps sous forme liquide, en utilisant des quantités équivalentes d'arginine et de glutamate. Il n'est donc pas nécessaire de lyophiliser les compositions comprenant du fibrinogène pour assurer leur stabilité à long terme. En outre, la Demanderesse a réussi à mettre au point des compositions comprenant du fibrinogène particulièrement adaptées aux injections, par exemple par voie intraveineuse, en minimisant le nombre et les quantités d'excipients. L'invention permet d'une manière générale de rationaliser et simplifier les procédés de production de ces différentes compositions, conduisant à un gain de temps et une réduction des coûts de production considérables. Avantageusement, lesdites compositions peuvent être exemptes d'autres excipients, notamment d'excipients connus pour leurs propriétés de préservation des lyophilisats, pour garantir une stabilité durant le stockage sous forme liquide prête à l'emploi. Un objet de l'invention est donc une composition pharmaceutique liquide comprenant :
- entre 10 et 30 g/L de fibrinogène ;
- entre 10 et 300 mM d'arginine ; et
- entre 10 et 300 mM de glutamate.
le pH de la composition étant compris entre 6 et 8.

Dans un mode de réalisation particulier, la composition est seulement constituée de fibrinogène, arginine et glutamate.

La composition selon l'invention comprend entre 10 g/L et 30 g/L de fibrinogène, de préférence entre 15 g/L et 25 g/L de fibrinogène, de préférence encore entre 15 g/L et 20 g/L de fibrinogène, et encore plus préférentiellement 15 g/L ou 20 g/L ou 25 g/L de fibrinogène.

Selon un mode de réalisation préféré, le fibrinogène est du fibrinogène humain, notamment obtenu à partir de plasma.

La concentration en arginine et la concentration en glutamate est comprise entre 10 et 300 mM, préférentiellement comprise entre 20 et 200 mM, plus préférentiellement comprise entre 30 et 100 mM, encore plus préférentiellement entre 50 et 80 mM.

Dans un mode de réalisation particulier, la concentration en arginine et la concentration en glutamate est de 10-80 mM. Une telle concentration en arginine et en glutamate est particulièrement adaptée pour les compositions comprenant du fibrinogène destinées à être injectées par voie intraveineuse.

Dans un mode de réalisation particulier, la concentration en arginine est comprise entre 10 et 300 mM, préférentiellement comprise entre 20 et 250 mM, plus préférentiellement comprise entre 50 et 250 mM.

Dans un autre mode de réalisation particulier, la concentration en arginine est avantageusement inférieure à 300 mM, de préférence inférieure à 250 mM, de préférence inférieure à 200 mM, encore plus préférentiellement inférieure à 150 mM. Une telle concentration en arginine est particulièrement adaptée pour éviter de trop augmenter l'osmolalité.

Dans un mode de réalisation particulier, la concentration en glutamate est avantageusement inférieure à 80 mM, de préférence inférieure à 70 mM, de préférence inférieure à 60 mM, encore plus préférentiellement inférieure à 50 mM. Une telle concentration en glutamate est particulièrement adaptée pour les compositions comprenant du fibrinogène destinées à être injectées par voie intraveineuse afin de minimiser les effets secondaires chez les patients.

Dans un autre mode de réalisation avantageux, la concentration en glutamate est comprise entre 10 et 80 mM, préférentiellement comprise entre 20 et 70 mM, plus préférentiellement comprise entre 30 et 60 mM. Une telle concentration en glutamate est particulièrement adaptée pour les compositions comprenant du fibrinogène destinées à être injectées en intraveineuse afin de minimiser les effets secondaires chez les patients.

Dans un mode de réalisation avantageux, la composition comprend en outre au moins un acide aminé choisi parmi l'alanine, l'asparagine, l'aspartate, la cystéine, la glutamine, la glycine, l'histidine, l'isoleucine, la leucine, la lysine, la méthionine, la phénylalanine, la proline, la sérine, la thréonine, le tryptophane, la tyrosine, la valine, seuls ou en combinaison, préférentiellement à une concentration d'environ 1-300 mM.

Dans un mode de réalisation particulier, la composition comprend en outre un acide aminé avantageusement choisi parmi la sérine, la proline et/ou l'isoleucine, préférentiellement à une concentration d'environ 1-300 mM.

Dans un mode de réalisation particulier, la composition comprend en outre un acide aminé polaire tel que la sérine, préférentiellement à une concentration d'environ 1-300 mM.

Dans un mode de réalisation particulier, la composition comprend en outre un acide aminé hydrophobe tel que la proline ou l'isoleucine, préférentiellement à une concentration d'environ 1-300 mM.

Dans un mode de réalisation particulier, la composition comprend en outre un surfactant, préférentiellement, du Pluronic®F68, du polysorbate 80 ou polysorbate 20 ou des sucres alkyles préférentiellement à une concentration d'environ 1-500 ppm.

Dans un mode de réalisation particulier, la composition comprend en outre du citrate trisodique, préférentiellement à une concentration de 1-15 mM

Dans un mode de réalisation particulier, la composition comprend en outre de l'albumine, à une concentration de 5-1000 ppm préférentiellement à une concentration de 5-500 ppm.

La composition selon l'invention est avantageusement une composition liquide prête à l'emploi.

La composition selon l'invention est avantageusement sous une forme adaptée pour une administration oculaire, nasale, intra-auriculaire, orale, sublinguale, pulmonaire, intrapéritonéale, intraveineuse, topique, percutanée, sous-cutanée, intra dermique, intra musculaire, transmuqueuse, vaginale, rectale, de préférence une administration intraveineuse.

### Description détaillée:

### Définitions

On entend par « fibrinogène » dans le cadre de l'invention, du fibrinogène humain. Il peut s'agir de fibrinogène humain fonctionnel, de séquence similaire ou identique à la séquence du fibrinogène humain normal, et toute fraction intermédiaire obtenue au cours du procédé de fabrication d'une composition comprenant du fibrinogène.

Le fibrinogène est une protéine constitué d'un dimère de trois chaines polypeptidiques dénommées alpha, beta et gamma. Le fibrinogène est donc un dimère et chaque monomère est composé de trois chaînes (alpha, bêta, gamma). La forme principale de fibrinogène a un poids moléculaire (PM) de 340 kDa. Le fibrinogène est formé de deux sous-unités identiques reliées par des ponts disulfures, donnant à la molécule une forme de fibre comportant 3 globules : un central (domaine E) et deux distaux (domaines D). La molécule entière contient 2964 acides aminés : 610 acides aminés pour la chaîne alpha (α), 461 acides aminés pour la chaîne beta (β), et 411 acides aminés pour la chaîne gamma (y).Le fibrinogène intervient dans l'hémostase primaire ainsi que dans la coagulation. Il est le plus souvent prescrit pour le traitement des complications associées à l'afibrinogénémie constitutionnelle ou sévère et les syndromes hémorragiques ou risques d'hémorragies associés à une hypofibrinogénémie.

Selon l'invention, on peut utiliser plusieurs sources de matière première contenant du fibrinogène. La composition comprenant du fibrinogène selon l'invention utilise ainsi une composition de fibrinogène, notamment de différentes sources. La composition de fibrinogène peut ainsi être issue de plasma sanguin, de surnageant de culture cellulaire ou de lait d'animaux transgéniques.

Dans un mode de réalisation particulier, la composition selon l'invention est une fraction plasmatique, de préférence une fraction plasmatique obtenue à partir de plasma sanguin prépurifié.

Par « fraction plasmatique obtenue à partir de plasma sanguin prépurifié», on entend toute partie ou sous-partie du plasma sanguin humain, ayant fait l'objet d'une ou plusieurs étapes de purification. Lesdites fractions plasmatiques incluent ainsi le cryosurnageant, le cryoprécipité de plasma (remis en suspension), la fraction I obtenue par fractionnement éthanolique (selon la méthode de Cohn ou de Kistler & Nitschmann), les éluats de chromatographies et les fractions non adsorbées des colonnes de chromatographie, y compris des chromatographies multicolonnes, et les filtrats.

Dans un mode de réalisation préféré de l'invention, la composition selon l'invention est issue d'un éluat de chromatographie ou d'une fraction non adsorbée de colonne de chromatographie, y compris de chromatographie multicolonnes.

Ainsi, dans un mode de réalisation préféré de l'invention, la composition selon l'invention est issue d'une fraction plasmatique obtenue à partir de cryosurnageant ou de cryoprécipité remis en suspension.

Selon l'invention, le « cryosurnageant », correspond à la phase liquide obtenue après décongélation de plasma congelé (cryoprécipitation). Notamment, le cryosurnageant peut être obtenu par congélation de plasma sanguin à une température comprise entre -10°C et - 40°C, puis décongélation douce à une température comprise entre 0°C et +6°C, préférentiellement entre 0°C et +1°C, suivie d'une centrifugation du plasma décongelé pour séparer le cryoprécipité et le cryosurnageant. Le cryoprécipité est un concentré en fibrinogène, fibronectine, facteur von Willebrand et facteur VIII, tandis que le cryosurnageant contient les facteurs du complément, les facteurs vitamine K dépendants tels que la protéine C, la protéine S, la protéine Z, le facteur II, le facteur VII, le facteur IX et le facteur X, du fibrinogène, les immunoglobulines et l'albumine.

Dans un mode de réalisation avantageux de l'invention, la composition selon l'invention peut être obtenue selon le procédé décrit par la Demanderesse dans la demande EP1739093 ou dans la demande WO2015/136217.

Dans un autre mode de réalisation particulier de l'invention, la composition selon l'invention provient de lait d'animaux transgéniques, par exemple obtenu selon la méthode décrite dans WO00/17234 ou dans WO00/17239.

Dans un mode de réalisation, la composition de l'invention peut être obtenue par le procédé comprenant les étapes suivantes :
- une étape de purification par chromatographie d'affinité;
- au moins une étape de sécurisation biologique; et
- une étape de formulation sous forme liquide.

Dans un mode de réalisation particulier de l'invention, l'étape de purification par chromatographie d'affinité se fait par chromatographie d'affinité utilisant des ligands d'affinité choisis parmi les anticorps, les fragments d'anticorps, les dérivés d'anticorps ou des ligands chimiques tels que des peptides, des peptides mimétiques, des peptoïdes, des nanofitines ou encore des ligands oligonucléotidiques tels que les aptamères.

Dans un mode de réalisation particulier de l'invention, la composition liquide, stable et comprenant du fibrinogène est obtenue selon le procédé comprenant les étapes suivantes :
- obtention d'une fraction de cryosurnageant de plasma sanguin,
- précipitation du cryosurnageant par 8 % d'éthanol afin d'obtenir une fraction enrichie en fibrinogène,
- purification de la fraction de plasma sanguin enrichie en fibrinogène après remise en suspension par séparation sur gel de chromatographie d'affinité utilisant préférentiellement des ligands d'affinité choisis parmi les anticorps, les fragments d'anticorps, les dérivés d'anticorps ou des ligands chimiques tels que des peptides, des peptides mimétiques, des peptoïdes, des nanofitines ou encore des ligands oligonucléotidiques tels que les aptamères,
- récupération de la fraction adsorbée purifiée comprenant du fibrinogène,
- éventuellement, ajout d'excipients pharmaceutiquement acceptables.

Dans un mode de réalisation particulier, le procédé comprend en outre une étape de conservation pendant au moins 3 mois à 5°C.

Dans un mode de réalisation particulier de l'invention, la chromatographie d'affinité utilisée est une matrice d'affinité avec des ligands de type fragment dérivé d'anticorps de lama telle que la matrice Fibrinogen CaptureSelect (Life Technologies).

De manière particulièrement avantageuse, la composition selon l'invention est dépourvue de protéases et/ou d'activateurs de la fibrinolyse.

Par « composition de fibrinogène dépourvue de protéases et/ou d'activateurs de la fibrinolyse», il est entendu que la composition de fibrinogène a subi une ou plusieurs étapes permettant l'élimination des protéases telles que la thrombine, la prothrombine, plasmine, plasminogène de telle manière que la quantité résiduelle de protéases et/ou d'activateurs de la fibrinolyse soit :
- très fortement réduite en comparaison à la solution de Fibrinogène pré-purifiée avant étape de chromatographie, et/ou
- nulle, et/ou
- inférieure aux seuils de détection des méthodes communément utilisées par l'homme du métier.

De manière avantageuse, le taux de prothrombine résiduel est inférieur à 5 µUI / mg de fibrinogène, le taux de plasminogène est inférieur à 15 ng / mg de fibrinogène.

Dans un mode de réalisation particulier de l'invention, la composition selon l'invention est donc dépourvue de protéases telles que la thrombine et/ou la plasmine ou leurs pro-enzymes correspondants prothrombine (facteur II de la coagulation) et/ou plasminogène, qui sont des zymogènes potentiellement activables.

Par « composition comprenant du fibrinogène », on entend une composition comprenant
- du fibrinogène humain
- éventuellement une ou plusieurs protéines copurifiées ou accompagnantes
- des excipients pharmaceutiquement acceptables

Selon l'invention, la ou les protéines copurifiées ou accompagnantes du fibrinogène peuvent consister en une ou plusieurs protéines plasmatiques. Par « protéine plasmatique », on entend selon l'invention toute protéine, et plus particulièrement toute protéine d'intérêt industriel ou thérapeutique, contenue dans le plasma sanguin. Les protéines du plasma sanguin englobent l'albumine, alpha/macroglobuline, antichymotrypsine, antithrombine, antitrypsine, Apo A, Apo B, Apo C, Apo D, Apo E, Apo F, Apo G, beta XIIa, C 1-inhibiteur, protéine C-réactive, C7, C1r, C1s, C2 C3, C4, C4bP, C5, C6, C1q, C8, C9, carboxypeptidase N, céruloplasmine, facteur B, facteur D, facteur H, facteur I, facteur IX, facteur V, facteur VII, facteur VIIa, facteur VIII, facteur X, facteur XI, facteur XII, facteur XIII, fibrinogène, fibronectine, haptoglobine, hemopexine, héparine cofacteur II, histidine rich GP, IgA, IgD, IgE, IgG, ITI, IgM, kininase II, kininogène HPM, lysozyme, PAI 2, PAI I, PCI, plasmine, inhibiteur de la plasmine, plasminogène, préalbumine, prékallicréine, properdine, protéase nexine INH, protéine C, protéine S, protéine Z, prothrombine, sérum amyloïde protéine (SAP), TFPI, thiol-protéinase, thrombomoduline, facteur tissulaire (TF), TPA, transcolabamine II, transcortine, transferrine, vitronectine, et le facteur de Willebrand. Notamment, les protéines plasmatiques englobent les protéines de la coagulation, c'est-à-dire les protéines plasmatiques impliquées dans la chaîne de réactions en cascade aboutissant à la formation d'un caillot sanguin. Les protéines de la coagulation englobent le facteur I (fibrinogène), le facteur II (prothrombine), le facteur V (proaccélérine), le facteur VII (proconvertine), le facteur VIII (facteur anti-hémophilique A), le facteur IX (facteur anti-hémophilique B), le facteur X (facteur Stuart), le facteur XI (facteur Rosenthal ou PTA), le facteur XII (facteur Hageman), le facteur XIII (facteur stabilisant la fibrine ou FSF), la PK (Prékallicréine) le KHPM (kininogène de haut poids moléculaire), le facteur III (thromboplastine ou facteur tissulaire), le cofacteur II de l'héparine (HCII), la protéine C (PC), la thrombomoduline (TM), la protéine S (PS), le facteur de Willebrand (Wf) et l'inhibiteur de la voie du facteur tissulaire (TFPI), ou encore les facteurs tissulaires.

Dans certains modes de réalisation, la protéine plasmatique consiste en une protéine de la coagulation à activité enzymatique. Les protéines de la coagulation à activité enzymatique englobent les formes activées du facteur II (prothrombine), facteur VII (proconvertine), facteur IX (facteur anti-hémophilique B), facteur X (facteur Stuart), facteur XI (Facteur Rosenthal ou PTA), facteur XII (facteur Hageman), facteur XIII (facteur stabilisant la fibrine ou FSF) et de la PK (Prékallicréine).

Le terme « excipient pharmaceutiquement acceptable » correspond à tout excipient avantageusement utilisable pour la formulation de protéines humaines, notamment aux substances choisies parmi les sels, les acides aminés, les sucres, les surfactants ou tout autre excipient.

Le terme « équimolaire » se réfère à une quantité identique ou équivalente en moles/L ou mmoles/L (M ou mM) entre plusieurs excipients, en particulier entre deux excipients, à un ratio entre les deux excipients compris entre 0,8 et 1,2, préférentiellement entre 0,9 et 1,1, encore plus préférentiellement environ égal à 1,0. En particulier, la composition selon l'invention comprend avantageusement une quantité équimolaire d'arginine et de glutamate, avec un ratio arginine/glutamate compris entre 0,8 et 1,2, préférentiellement entre 0,9 et 1,1, encore plus préférentiellement environ égal à 1,0 ; ou un ratio glutamate/arginine compris entre 0,8 et 1,2, préférentiellement entre 0,9 et 1,1, encore plus préférentiellement environ égal à 1,0.

Le terme « stable » correspond à la stabilité physique et/ou chimique de la composition comprenant le fibrinogène. Le terme « stabilité physique » se réfère à la réduction ou l'absence de formation d'agrégats insolubles ou solubles des formes dimériques, oligomériques ou polymériques du fibrinogène, à la réduction ou l'absence de formation de précipité, ainsi qu'à la réduction ou l'absence de toute dénaturation structurale de la molécule.

Le terme « stabilité chimique » se réfère à la réduction ou l'absence de toute modification chimique de la composition comprenant le fibrinogène pendant le stockage, à l'état liquide, dans des conditions accélérées.

La stabilité d'une composition comprenant du fibrinogène peut s'évaluer par différentes méthodes, notamment par des méthodes de test de stabilité accéléré ou par des méthodes de mise en stabilité long terme.

Les méthodes de test de stabilité accéléré comprennent notamment des tests de stress mécaniques par chauffage.

Dans un mode de réalisation, la stabilité de la composition comprenant du fibrinogène est mesurée après un stress thermique (heating stress) par chauffage en bain thermostaté à 37°C, par exemple par mesure à T0, T+7jours et T+14jours. Ensuite la mesure des particules en solution de tailles supérieure au seuil de détection par l'œil humain (environ 50µm) est effectuée notamment par inspection visuelle à l'aide par exemple d'une mireuse pharmacopée européenne (opalescence, formation de particules), par mesure de la turbidité au moyen d'un spectrophotomètre mesurant l'absorbance ou densité optique à 400 nm.

Un test de stabilité long terme peut être effectué en différentes conditions de température, d'humidité, et de lumière. Préférentiellement, dans le cadre de la présente invention, le test de stabilité peut durer au minimum 1 semaine, préférentiellement au moins 1 mois, préférentiellement au moins 2 mois, préférentiellement au moins 3 mois, préférentiellement au moins 4 mois, préférentiellement au moins 5 mois, plus préférentiellement au moins 6 mois.

Typiquement, la mesure des paramètres de stabilité, tel que définis ci-après, ont lieu
- avant la mise en test de stabilité d'une composition comprenant du fibrinogène ; on peut alors parler de taux initial; et
- pendant ou à l'issue dudit test de stabilité,
étant entendu que ledit test de stabilité peut durer au minimum 1 semaine, préférentiellement au moins 1 mois, préférentiellement au moins 2 mois, préférentiellement au moins 3 mois, préférentiellement au moins 4 mois, préférentiellement au moins 5 mois, plus préférentiellement au moins 6 mois.

Les méthodes d'analyse après mise en stabilité long terme comprennent notamment des analyses par inspection visuelle à l'aide notamment d'une mireuse pharmacopée européenne (opalescence, formation de particules), par mesure de la turbidité au moyen d'un spectrophotomètre mesurant l'absorbance ou densité optique à 400 nm, permettant d'évaluer la présence ou non de dégradation du produit par la détection de trouble dans la solution.

Dans un mode de réalisation, la stabilité de la composition comprenant du fibrinogène est définie par la mesure du taux de monomères conservés pendant le test de stabilité au moyen de la méthode High Pressure Size Exclusion Chromatography (HPSEC). Ces méthodes sont bien connues de l'homme du métier.

Une composition de fibrinogène est avantageusement considérée comme stable si la quantité de monomères de fibrinogène conservés pendant la mise en test de stabilité est supérieure à 50%, préférentiellement supérieure à 60%, préférentiellement supérieure à 70%, préférentiellement supérieure à 80%, préférentiellement supérieure à 90%, préférentiellement supérieure à 95% du taux initial de monomères de fibrinogène.

Plus préférentiellement, la quantité de monomères de fibrinogène conservés pendant le test de stabilité est supérieure à 70% du taux initial de monomères de fibrinogène.

Par "taux initial de monomère de fibrinogène", on entend le taux de monomère observé avant la mise en test de stabilité. Typiquement, la quantité de monomère de fibrinogène est mesurée avant la mise en test de stabilité et pendant ou à l'issue dudit test de stabilité.

Alternativement, une composition de fibrinogène est considérée comme stable si la variation de quantité de monomères de fibrinogène durant le test de stabilité est inférieure à 20 %, préférentiellement inférieure à 10%, préférentiellement inférieure à 5%, préférentiellement inférieure à 1%.

Dans un autre mode de réalisation, la stabilité de la composition comprenant du fibrinogène est définie par la mesure du taux de polymères de fibrinogène formés pendant la mise en test de stabilité au moyen de HPSEC. Les polymères de fibrinogènes sont des polymères comprenant au moins 2 chaines polypeptidiques alpha, 2 chaines polypeptidiques beta et 2 chaines polypeptidiques gamma du fibrinogène. Ce terme inclue également les trimères de fibrinogène.

Une composition de fibrinogène est avantageusement considérée comme stable si la quantité de polymères de fibrinogène formés pendant la mise en test de stabilité est inférieure à 10%, préférentiellement inférieure à 20%, préférentiellement inférieure à 30%, préférentiellement inférieure à 40%, préférentiellement inférieure à 50% par rapport au taux initial de polymères de fibrinogène. Typiquement, le taux initial de polymères de fibrinogène correspond à l'ensemble des formes polymériques (trimères et plus) du fibrinogène avant la mise en test de stabilité.

Plus préférentiellement, la quantité de polymères de fibrinogène formés pendant la mise en test de stabilité est inférieure à 30%. Typiquement, la quantité de polymères de fibrinogène est mesurée avant la mise en test de stabilité et pendant ou à l'issue dudit test de stabilité.

Alternativement, une composition de fibrinogène est considérée comme stable si la variation de quantité de polymères de fibrinogène durant le test de stabilité est inférieure à 20%, préférentiellement inférieure à 10%, préférentiellement inférieure à 5%, préférentiellement inférieure à 1%.

Dans un autre mode de réalisation, la stabilité de la composition comprenant du fibrinogène est évaluée par la mesure de l'activité coagulable du fibrinogène rapportée à la mesure antigénique du fibrinogène (aussi appelée activité spécifique). De manière particulièrement avantageuse, la composition de fibrinogène stable présente un ratio fibrinogène coagulable / fibrinogène antigène supérieur à 0,5; préférentiellement supérieur à 0,6; supérieur à 0,7; supérieur à 0,8; supérieur à 0,9; encore plus préférentiellement environ égal à 1,0.

Par « fibrinogène coagulable » on entend la mesure du fibrinogène fonctionnel par une technique de coagulation, déterminée selon la méthode de von Clauss. L'activité coagulable est exprimée eu g/L de solution de fibrinogène. Cette technique est connue de l'homme du métier qui peut se référer à la publication Von Clauss, A. (1957) Gerinnungsphysiologische schnellmethode zur bestimmung des fibrinogens. Acta Haematologica, 17, 237-246.

Par « fibrinogène antigène » on entend la quantité de fibrinogène, qu'il soit actif ou non, mesurée par méthode néphélémétrique. La quantité de fibrinogène antigénique est exprimée en g/L.

La stabilité de la composition comprenant du fibrinogène est également évaluée par la mesure en SDS PAGE de la conservation des chaînes alpha, beta et gamma de fibrinogène, préférentiellement avant et après un test de stabilité comme défini dans le cadre de la présente invention. Ainsi, une composition de fibrinogène est avantageusement considérée comme stable si :
- l'ensemble des chaînes alpha est conservée à au moins 50%, préférentiellement au moins 60%, préférentiellement au moins 70%, préférentiellement au moins 80%, préférentiellement au moins 90%; plus préférentiellement conservée à environ 100%, et/ou
- l'ensemble des chaînes beta est conservée à au moins 50%, préférentiellement au moins 60%, préférentiellement au moins 70%, préférentiellement au moins 80%, préférentiellement au moins 90%; plus préférentiellement conservée à environ 100%, et/ou
- l'ensemble des chaînes gamma est conservée à au moins 50%, préférentiellement au moins 60%, préférentiellement au moins 70%, préférentiellement au moins 80%, préférentiellement au moins 90%; plus préférentiellement conservée à environ 100%.

La stabilité de la composition comprenant du fibrinogène est également définie par la mesure de la turbidité au moyen de la spectrophotométrie UV à 400nm. En effet, la turbidité reflète la quantité en matière qui trouble la solution. Une composition de fibrinogène est avantageusement considérée comme stable si la turbidité mesurée après le test de stabilité comme défini dans la présente invention est comparable à la turbidité mesurée avant stabilité.

Avantageusement, la turbidité mesurée après la mise en test de stabilité correspond à moins de 130%, moins de 120%, moins de 110% ; avantageusement correspond à 100% de la turbidité mesurée avant stabilité.

Dans un mode de réalisation avantageux de l'invention, la composition comprenant du fibrinogène est stable pendant au moins 1 mois, au moins 2 mois, au moins 3 mois, au moins 4 mois, au moins 5 mois, au moins 6 mois à entre 2°C et 8°C.

Dans un mode de réalisation avantageux de l'invention, la composition comprenant du fibrinogène est stable pendant 6 mois entre 2°C et 8°C.

Par « composition de fibrinogène sous forme liquide » on entend une composition comprenant du fibrinogène en solution, préférentiellement qui n'a pas été soumise à une étape de lyophilisation, dessiccation, déshydratation, spray drying ou séchage, et qui n'a donc pas besoin d'être reconstituée avant utilisation.

Dans le contexte de l'invention, l'expression « compris entre x et y » signifie que les valeurs x et y sont incluses.

### Formulations :

La composition selon l'invention comprend entre 10 g/L et 30 g/L de fibrinogène (référencées ci-après compositions fibrinogène 10-30 g/L), de préférence entre 15 g/L et 25 g/L de fibrinogène, de préférence encore entre 15 g/L et 20 g/L de fibrinogène, et encore plus préférentiellement 15 g/L ou 20 g/L ou 25 g/L de fibrinogène.

Dans le contexte de la présente invention, les concentrations s'entendent au niveau de la composition finale, prête à l'emploi. Les concentrations sont déterminées vis à vis des compositions sous forme liquide.

De manière particulièrement avantageuse, la Demanderesse a mis en évidence qu'il est possible d'obtenir des compositions liquides comprenant 10-30 g/L de fibrinogène particulièrement stables dans le temps en utilisant un minimum d'excipients. Ainsi, selon l'invention, les compositions liquides de fibrinogène 10-30 g/L comprennent avantageusement entre 10 et 300 mM d'arginine, de manière préférée entre 20 et 200 mM, et entre 10 et 300 mM de glutamate, de manière préférée entre 20 et 200 mM.

Dans un mode de réalisation particulier, la concentration en arginine est comprise entre 10 et 300 mM, préférentiellement comprise entre 20 et 250 mM, plus préférentiellement comprise entre 50 et 250 mM.

Dans un autre mode de réalisation particulier, la concentration en arginine est avantageusement inférieure à 300 mM, de préférence inférieure à 250 mM, de préférence inférieure à 200 mM, encore plus préférentiellement inférieure à 150 mM. Une telle concentration en arginine est particulièrement adaptée pour éviter de trop augmenter l'osmolalité.

Dans un mode de réalisation particulier, la concentration en glutamate est avantageusement inférieure à 80 mM, de préférence inférieure à 70 mM, de préférence inférieure à 60 mM, encore plus préférentiellement inférieure à 50 mM. Une telle concentration en glutamate est particulièrement adaptée pour les compositions comprenant du fibrinogène destinées à être injectées par voie intraveineuse afin de minimiser les effets secondaires chez les patients.

Dans un autre mode de réalisation avantageux, la concentration en glutamate est comprise entre 10 et 80 mM, préférentiellement comprise entre 20 et 70 mM, plus préférentiellement comprise entre 30 et 60 mM. Une telle concentration en glutamate est particulièrement adaptée pour les compositions comprenant du fibrinogène destinées à être injectées en intraveineuse afin de minimiser les effets secondaires chez les patients.

Dans un autre mode de réalisation particulier, la composition liquide de fibrinogène 10-30 g/L comprend de l'arginine et du glutamate en quantités équimolaires. La Demanderesse a mis en évidence de manière surprenante que l'ajout en quantités équimolaires de glutamate et d'arginine permet avantageusement de stabiliser la formulation sous forme liquide tout en maintenant une bonne tolérance vis-à-vis des patients. L'effet de stabilisation est proportionnel à l'augmentation des quantités de glutamate et d'arginine. La Demanderesse a néanmoins mis en évidence un effet optimum entre 10 et 80 mM, en effet, au-delà de 80 mM, la composition, bien que stable, présente un taux de glutamate susceptible d'entraîner des effets secondaires chez les patients.

De même, dans un mode de réalisation particulier, les compositions de fibrinogène 10-30 g/L comprennent avantageusement entre 1 et 500 ppm de surfactant, préférentiellement entre 20 et 400 ppm de surfactant, préférentiellement encore entre 50 et 400 ppm de surfactant, plus préférentiellement entre 150 et 250 ppm. Le surfactant utilisé dans la composition selon l'invention est avantageusement choisi parmi les surfactants non ioniques, de préférence les polysorbates et notamment parmi le polysorbate 80 (ou Tween®80 qui est du polyoxyéthylènesorbitanne-monooléate), et le polysorbate 20 (ou Tween®20 qui est du polyoyéthylènesorbitanne-monolaurate). Eventuellement, le surfactant peut être choisi parmi les poloxamères, polyoxyéthylène alkyl éthers, un bloc co-polymères d'éthylène/polypropylène, les alkyl glucosides ou sucres alkyles, et le Pluronic®F68 (polyéthylènepolypropylène glycol). Les surfactants peuvent également être combinés entre eux. De manière avantageuse, le surfactant permet de stabiliser les interactions entre les molécules sous formes liquide.

Selon un mode de réalisation particulier de l'invention, la composition comprend en outre au moins un acide aminé choisi parmi l'alanine, l'asparagine, l'aspartate, la cystéine, la glutamine, la glycine, l'histidine, l'isoleucine, la leucine, la lysine, la méthionine, la phénylalanine, la proline, la sérine, la thréonine, le tryptophane, la tyrosine, la valine, seuls ou en combinaison, préférentiellement à une concentration d'environ 1-300 mM.

Selon un mode de réalisation particulier de l'invention, la composition comprend en outre un acide aminé avantageusement choisi parmi la sérine, la proline et/ou l'isoleucine, préférentiellement à une concentration d'environ 1-300 mM. De manière avantageuse, l'acide aminé stabilise la composition selon l'invention sous forme liquide.

Dans un mode de réalisation particulier, la composition comprend en outre un acide aminé polaire tel que la sérine, préférentiellement à une concentration d'environ 1-300 mM.

Selon un mode de réalisation particulier de l'invention, la composition contient également au moins un acide aminé hydrophobe choisi parmi la leucine, l'alanine, la phénylalanine, le tryptophane, la valine, la méthionine, l'isoleucine, la proline, la cystéine et/ou la glycine. De manière préférée, l'acide aminé hydrophobe est choisi parmi la proline et/ou l'isoleucine. De manière avantageuse, l'acide aminé hydrophobe stabilise la composition selon l'invention sous forme liquide.

Selon un mode de réalisation particulier de l'invention, la composition contient également un tampon, en particulier un tampon histidine, un tampon phosphate, du Tris-HCl ou du citrate trisodique, préférentiellement du citrate trisodique, préférentiellement à une concentration comprise entre 1 et 15 mM, plus préférentiellement à une concentration comprise entre 7 et 10 mM, encore plus préférentiellement à une concentration comprise entre 8 et 9 mM.

Selon un mode de réalisation particulier de l'invention, la composition contient également de l'albumine à une concentration comprise entre 5 et 1000 ppm, préférentiellement à une concentration comprise entre 5 et 500 ppm. De manière avantageuse, l'albumine présente dans la composition de l'invention est de l'albumine humaine plasmatique ou recombinante.

De manière avantageuse, la Demanderesse a mis en évidence que de telles compositions présentent une osmolalité particulièrement adaptée à l'administration par injection, notamment par voie intraveineuse, et cela sans ajout en nombre et/ou quantités d'excipients. Ainsi, l'invention propose des compositions de fibrinogène 15-25 g/L ou 15-20 g/L présentant une osmolalité mesurée comprise entre 250 et 550 mOsm/kg environ. Dans le contexte de l'invention, et sauf mention contraire, l'osmolalité de la composition s'entend de l'osmolalité mesurée dans ladite composition.

L'osmolalité est avantageusement mesurée à l'aide d'un osmomètre calibré avec des solutions étalons, et notamment selon la méthode préconisée par la Pharmacopée Européenne, (Pharmacopée Européenne 5.0 de 2005 -01/2005:2.2.35.). Bien entendu, toute autre méthode de mesure de l'osmolalité peut être utilisée.

Dans un mode de réalisation particulier, les seuls excipients de la composition de fibrinogène selon l'invention, sont l'arginine et le glutamate. Une telle formulation permet une bonne stabilisation des compositions liquides de fibrinogène et une réduction des durées et des coûts de préparation à l'échelle industrielle grâce à la présence d'un nombre et d'une quantité minimums efficaces d'excipients. Avantageusement, une telle composition présente une osmolalité compatible avec l'administration par injection, notamment par voie intraveineuse.

Dans un autre mode de réalisation particulier, les seuls excipients de la composition de fibrinogène selon l'invention, sont l'arginine, le glutamate et le surfactant.

Dans un autre mode de réalisation particulier, les seuls excipients de la composition de fibrinogène selon l'invention, sont l'arginine, le glutamate et au moins un autre acide aminé.

Dans un autre mode de réalisation particulier, les seuls excipients de la composition de fibrinogène selon l'invention, sont l'arginine, le glutamate et au moins un acide aminé hydrophobe.

Dans un autre mode de réalisation particulier, les seuls excipients de la composition de fibrinogène selon l'invention, sont l'arginine, le glutamate, le surfactant et au moins un autre acide aminé.

Dans un autre mode de réalisation particulier, les seuls excipients de la composition de fibrinogène selon l'invention, sont l'arginine, le glutamate, le surfactant et au moins un acide aminé hydrophobe.

Dans un autre mode de réalisation particulier, les seuls excipients de la composition de fibrinogène selon l'invention, sont l'arginine, le glutamate et de l'albumine humaine.

Dans un mode de réalisation particulier, la composition consiste essentiellement en du fibrinogène, de l'arginine, du glutamate, en ce sens que tout autre excipient qui pourrait être présent ne le serait qu'à l'état de trace.

Dans un autre mode de réalisation particulier de l'invention, la composition selon l'invention est dépourvue d'ions divalents, notamment d'ions métalliques divalents, en particulier de calcium et/ou de sodium.

De manière particulièrement avantageuse, la composition selon l'invention est dépourvue d'excipients connus pour leur rôle de stabilisation des lyophilisats tels que le chlorure de sodium, et/ou le CarboxyMethylDextran (CMD).

Dans un autre mode de réalisation particulier de l'invention, la composition selon l'invention est dépourvue d'albumine.

Dans un autre mode de réalisation particulier de l'invention, la composition selon l'invention est dépourvue de sucres.

Dans un mode de réalisation particulier de l'invention, la composition de fibrinogène selon l'invention est dépourvue d'inhibiteurs de protéases et/ou d'anti-fibrinolytiques.

Par « inhibiteurs de protéases et/ou anti-fibrinolytiques», on entend toute molécule à activité antiprotéasique, notamment toute molécule à activité inhibitrice de sérine protéase et/ou anti-fibrinolytique, en particulier toute molécule à activité inhibitrice de thrombine et/ou anti-plasmine, en particulier l'hirudine, la benzamidine, l'aprotinine, le florure de phénylméthylsulfonyle (PMSF), la pepstatine, la leupeptine, l'antithrombine III associée ou non avec de l'héparine, l'alpha 2 macroglobuline, l'alpha 1 antitrypsine, l'acide hexanoïque ou epsilon aminocaproïque, l'acide tranéxamique, l'alpha 2 antiplasmine, le DiosoPropylfluoroPhosphate (DSP), l'antichimotrypsine.

Dans un mode de réalisation particulier de l'invention, la composition de fibrinogène selon l'invention est dépourvue d'hirudine et/ou de benzamidine et/ou d'aprotinine et/ou de PMSF et/ou de pepstatine et/ou de leupeptine et/ou d'antithrombine III associée ou non avec de l'héparine et/ou d'alpha 2 macroglobuline et/ou d'alpha 1 antitrypsine et/ou d'acide hexanoïque et/ou epsilon aminocaproïque et/ou d'acide tranéxamique et/ou d'alpha 2 antiplasmine.

Dans un mode de réalisation particulier de l'invention, la composition selon l'invention ne comprend pas d'autres protéines copurifiées, avantageusement pas de FXIII et/ou de fibronectine.

Dans un autre mode de réalisation particulier de l'invention, la composition de fibrinogène selon l'invention peut également comprendre une ou plusieurs protéines accompagnantes, éventuellement copurifiées. Dans un mode de réalisation particulier de l'invention, la composition selon l'invention comprend avantageusement du FXIII.

Avantageusement, la composition selon l'invention est dépourvue de fibrine.

Dans un autre mode de réalisation particulier, la composition consiste essentiellement en du fibrinogène, de l'arginine, du glutamate, éventuellement un surfactant, de préférence du tween 80, éventuellement un acide aminé hydrophobe, préférentiellement de la proline et/ou de l'isoleucine, et éventuellement du tampon tel que le citrate trisodique, en ce sens que tout autre excipient qui pourrait être présent ne le serait qu'à l'état de trace.

Selon l'invention, le pH final de la composition est avantageusement compris entre 6 et 8. Préférentiellement, le pH est d'environ 6,0-7,5, encore plus préférentiellement entre 6,0-7,0. Un pH de 6,0-7,0 donne en effet des résultats particulièrement satisfaisants en termes de stabilité liquide dans le temps en permettant à la fois de limiter les phénomènes d'agrégation et de dégradation. Le pH final s'entend du pH de la composition après formulation, c'est-à-dire dans la composition prête à l'emploi. Sauf mention contraire, dans la présente description, le pH de la composition désigne le pH final.

Une composition de fibrinogène 10-30 g/L préférée selon l'invention comprend :
- 10-300 mM d'arginine
- 10-300 mM de glutamate
- éventuellement 1-15 mM de tampon, en particulier de citrate trisodique
- éventuellement 1-300 mM d'un autre acide aminé, en particulier 1-300 mM de proline et/ou 1-300 mM d'isoleucine et/ou 1-300 mM de sérine
- éventuellement 1-500 ppm de surfactant, préférentiellement 20-400 ppm, de manière encore préférée 150-250 ppm
- éventuellement 5-1000 ppm d'albumine humaine
le pH de la composition étant de 6,0-8,0.

Une composition de fibrinogène 10-30 g/L préférée selon l'invention comprend :
- 20-200 mM d'arginine
- 20-200 mM de glutamate
- éventuellement 7-10 mM de tampon, en particulier de citrate trisodique
- éventuellement 1-100 mM d'un autre acide aminé, en particulier 1-100 mM de proline et/ou 1-100 mM d'isoleucine et/ou 1-100 mM de sérine
- éventuellement 20-400 ppm de surfactant
- éventuellement 5-500 ppm d'albumine humaine
le pH de la composition étant de 6,0-8,0.

Une composition de fibrinogène 15-25 g/L préférée selon l'invention comprend :
- 150 mM d'arginine
- 80 mM de glutamate
- 8,5 mM de tampon, en particulier de citrate trisodique
- 200 ppm de surfactant, préférentiellement de polysorbate80, de polysorbate20 ou de Pluronic®F68
- éventuellement 1-100 mM d'un autre acide aminé, en particulier 1-100 mM de proline et/ou 1-100 mM d'isoleucine et/ou 1-100 mM de sérine
- éventuellement 5-500 ppm d'albumine humaine
le pH de la composition étant de 6,0-7,0.

Une composition de fibrinogène 15-25 g/L préférée selon l'invention comprend :
- 10-300 mM d'arginine
- 10-300 mM de glutamate
le pH de la composition étant de 6,0-8,0.

Une composition de fibrinogène 15-20 g/L préférée selon l'invention comprend :
- 10-300 mM d'arginine
- 10-300 mM de glutamate
le pH de la composition étant de 6,0-8,0.

Une autre composition de fibrinogène 15-25 g/L préférée selon l'invention comprend :
- 10-300 mM d'arginine
- 10-300 mM de glutamate
- 1-15 mM de tampon, en particulier de citrate trisodique
le pH de la composition étant de 6,0-8,0.

Une autre composition de fibrinogène 15-20 g/L préférée selon l'invention comprend :
- 10-300 mM d'arginine
- 10-300 mM de glutamate
- 1-15 mM de citrate trisodique
le pH de la composition étant de 6,0-8,0.

Une autre composition de fibrinogène 15-25 g/L préférée selon l'invention comprend :
- 10-300 mM d'arginine
- 10-300 mM de glutamate
- 1-300 mM d'un autre acide aminé
le pH de la composition étant de 6,0-8,0.

Une autre composition de fibrinogène 15-20 g/L préférée selon l'invention comprend :
- 10-300 mM d'arginine
- 10-300 mM de glutamate
- 1-300 mM d'acide aminé hydrophobe
le pH de la composition étant de 6,0-8,0.

Une autre composition de fibrinogène 15-25 g/L préférée selon l'invention comprend :
- 10-300 mM d'arginine
- 10-300 mM de glutamate
- 1-300 mM d'un autre acide aminé
- 1-15 mM de tampon, en particulier de citrate trisodique
le pH de la composition étant de 6,0-8,0.

Une autre composition de fibrinogène 15-20 g/L préférée selon l'invention comprend :
- 10-300 mM d'arginine
- 10-300 mM de glutamate
- 1-300 mM d'acide aminé hydrophobe
- 1-15 mM de citrate trisodique
le pH de la composition étant de 6,0-8,0.

Une autre composition de fibrinogène 15-25 g/L préférée selon l'invention comprend :
- 10-300 mM d'arginine
- 10-300 mM de glutamate
- 1-300 mM de proline et/ou d'isoleucine et/ou de sérine
le pH de la composition étant de 6,0-8,0.

Une autre composition de fibrinogène 15-20 g/L préférée selon l'invention comprend :
- 10-300 mM d'arginine
- 10-300 mM de glutamate
- 1-300 mM de proline et/ou d'isoleucine et/ou de sérine
le pH de la composition étant de 6,0-8,0.

Une autre composition de fibrinogène 15-20 g/L préférée selon l'invention comprend :
- 10-300 mM d'arginine
- 10-300 mM de glutamate
- 1-300 mM de proline et/ou d'isoleucine
le pH de la composition étant de 6,0-8,0.

Une autre composition de fibrinogène 15-20 g/L préférée selon l'invention comprend :
- 10-300 mM d'arginine
- 10-300 mM de glutamate
- 1-500 ppm de surfactant, préférentiellement 50-400 ppm, de manière encore préférée 150-250 ppm
le pH de la composition étant de 6,0-8,0.

Une autre composition de fibrinogène 15-20 g/L préférée selon l'invention comprend :
- 10-300 mM d'arginine
- 10-300 mM de glutamate
- 1-500 ppm de surfactant, préférentiellement 50-400 ppm, de manière encore préférée 150-250 ppm
- 1-15 mM de citrate trisodique
le pH de la composition étant de 6,0-8,0.

Une autre composition de fibrinogène 15-20 g/L préférée selon l'invention comprend :
- 10-300 mM d'arginine
- 10-300 mM de glutamate
- 1-500 ppm de polysorbate 80, préférentiellement 50-400 ppm, de manière encore préférée 150-250 ppm
le pH de la composition étant de 6,0-8,0.

Une autre composition de fibrinogène 15-20 g/L préférée selon l'invention comprend :
- 10-300 mM d'arginine
- 10-300 mM de glutamate
- 1-300 mM d'acide aminé hydrophobe
- 1-500 ppm de surfactant, préférentiellement 50-400 ppm, de manière encore préférée 150-250 ppm
- 1-15 mM de citrate trisodique
le pH de la composition étant de 6,0-8,0.

Une autre composition de fibrinogène 15-20 g/L préférée selon l'invention comprend :
- 10-300 mM d'arginine
- 10-300 mM de glutamate
- 1-300 mM de proline et/ou d'isoleucine
- 1-500 ppm de polysorbate 80, préférentiellement 50-400 ppm, de manière encore préférée 150-250 ppm
- éventuellement 1-15 mM de citrate trisodique
le pH de la composition étant de 6,0-8,0.

L'osmolalité mesurée de cette composition de fibrinogène est avantageusement d'environ 225-715 mOsm/kg.

De manière surprenante et avantageuse, la demanderesse a mis en évidence qu'une concentration en arginine d'environ 10-80 mM, +/- 10% combinée à une concentration en glutamate d'environ 10-80 mM, +/- 10%, est suffisante pour maintenir la composition de fbrinogène 15-20 g/L liquide stable tout en maintenant une osmolalité comprise entre 225 et 500 mOsm/kg dans la composition, alors que des concentrations supérieures auraient été attendues pour garantir la stabilité liquide, augmentant parallèlement l'osmolalité desdites compositions. Or, une trop forte osmolalité peut être à l'origine d'une déshydratation des cellules (sortie de l'eau intracellulaire vers le milieu extracellulaire) préjudiciable au patient.

De manière avantageuse, la composition selon l'invention possède une pureté supérieure ou égale à 70%, de manière préférée supérieure ou égale à 75%, 80%, 85%, 90%, 95%, 96%, 97%, 98%, 99%.

La composition de fibrinogène selon l'invention, sous forme liquide et après un stockage durant au moins une période de 6 mois entre 2°C et 8°C présente avantageusement une quantité de monomères de fibrinogène conservés pendant la mise en test de stabilité supérieure à 50%, préférentiellement supérieure à 60%, préférentiellement supérieure à 70%, préférentiellement supérieure à 80%, préférentiellement supérieure à 90%, préférentiellement supérieure à 95% du taux initial de monomères de fibrinogène.

Plus préférentiellement, la composition de fibrinogène selon l'invention, sous forme liquide et après un stockage durant au moins une période de 6 mois à 5°C ± 3°C présente avantageusement une quantité de monomères de fibrinogène conservés pendant la mise en test de stabilité supérieure à 70% du taux initial de monomères de fibrinogène.

Alternativement, la composition de fibrinogène selon l'invention, sous forme liquide, présente avantageusement une variation de quantité de monomères de fibrinogène durant le test de stabilité inférieure à 20 %, préférentiellement inférieure à 10%, préférentiellement inférieure à 5%, préférentiellement inférieure à 1%.

La composition de fibrinogène selon l'invention, sous forme liquide et après un stockage durant au moins une période de 6 mois entre 2°C et 8°C présente avantageusement une quantité de polymères de fibrinogène formés pendant la mise en test de stabilité inférieure à 10%, préférentiellement inférieure à 20%, préférentiellement inférieure à 30%, préférentiellement inférieure à 40%, préférentiellement inférieure à 50% par rapport au taux initial de polymères de fibrinogène. Typiquement, le taux initial de polymères de fibrinogène correspond à l'ensemble des formes polymériques (trimères et plus) du fibrinogène avant la mise en test de stabilité.

Plus préférentiellement, la composition de fibrinogène selon l'invention, sous forme liquide et après un stockage durant au moins une période de 6 mois entre 2°C et 8°C présente avantageusement une quantité de polymères de fibrinogène formés pendant la mise en test de stabilité inférieure à 30%. Typiquement, la quantité de polymères de fibrinogène est mesurée avant la mise en test de stabilité et pendant ou à l'issue dudit test de stabilité. Alternativement, la composition de fibrinogène selon l'invention, sous forme liquide, présente avantageusement une variation de quantité de polymères de fibrinogène durant le test de stabilité est inférieure à 20%, préférentiellement inférieure à 10%, préférentiellement inférieure à 5%, préférentiellement inférieure à 1%.

La composition de fibrinogène selon l'invention, sous forme liquide et après un stockage durant au moins une période de 6 mois entre 2°C et 8°C présente avantageusement un ratio fibrinogène coagulable / fibrinogène antigène supérieur à 0,5; préférentiellement supérieur à 0,6; supérieur à 0,7; supérieur à 0,8; supérieur à 0,9; encore plus préférentiellement environ égal à 1,0.

La composition de fibrinogène selon l'invention, sous forme liquide et après un stockage durant au moins une période de 6 mois entre 2°C et 8°C présente avantageusement
- une conservation de l'ensemble des chaînes alpha à au moins 50%, préférentiellement au moins 60%, préférentiellement au moins 70%, préférentiellement au moins 80%, préférentiellement au moins 90%; plus préférentiellement conservée à environ 100%, et/ou
- une conservation de l'ensemble des chaînes beta à au moins 50%, préférentiellement au moins 60%, préférentiellement au moins 70%, préférentiellement au moins 80%, préférentiellement au moins 90%; plus préférentiellement conservée à environ 100%, et/ou
- une conservation de l'ensemble des chaînes gamma à au moins 50%, préférentiellement au moins 60%, préférentiellement au moins 70%, préférentiellement au moins 80%, préférentiellement au moins 90%; plus préférentiellement conservée à environ 100%.

La composition de fibrinogène selon l'invention, sous forme liquide et après un stockage durant au moins une période de 6 mois entre 2°C et 8°C présente avantageusement une turbidité mesurée après la mise en test de stabilité correspondant à moins de 130%, moins de 120%, moins de 110% ; avantageusement correspondant à 100% de la turbidité mesurée avant stabilité.

Les compositions de l'invention peuvent être des compositions pharmaceutiques, c'est-à-dire adaptées à un usage thérapeutique. Les compositions pharmaceutiques de l'invention sont ainsi utiles comme médicaments, notamment afin de traiter les hypo-fibrinogénémies, dys-fibrinogénémies ou a-fibrinogénémies constitutionnelles chez les patients présentant une hémorragie spontanée ou post-traumatique, ou comme traitement complémentaire dans la prise en charge d'une hémorragie sévère incontrôlée dans le cadre d'une hypofibrinogénémie acquise.

Selon l'invention, on peut utiliser plusieurs sources de matière première contenant du fibrinogène. La composition de fibrinogène peut ainsi être issue de plasma sanguin autrement dénommées fractions plasmatiques, de surnageant de culture cellulaire ou de lait d'animaux transgéniques.

Dans un mode de réalisation préféré, la composition de l'invention n'a subi aucune étape préalable de lyophilisation, dessiccation, déshydratation ou séchage.

Dans un mode de réalisation préféré, la composition de l'invention n'a subi aucune étape préalable de reconstitution d'un lyophilisat.

Les compositions selon l'invention peuvent être avantageusement soumises à au moins une méthode d'élimination ou d'inactivation des agents infectieux

Une inactivation virale comprend souvent un traitement avec des produits chimiques, par exemple par solvant, et/ou détergent et/ou par la chaleur (pasteurisation et/ou chauffage) et/ou par irradiation (Gamma et/ou par les UVC) et/ou par traitement pH (traitement à pH acide).

De préférence, l'inactivation virale consiste en une étape de traitement par chauffage ou par un traitement par solvant et détergent. Le traitement par solvant et détergent (appelé généralement traitement Solvant/Détergent ou S/D) comprend notamment le traitement par le tri-n-butylphosphate (TnBP) et/ou un détergent qui est choisi parmi le Triton X-100, le Tween (de préférence Tween 80), le cholate de sodium et le 2-[4-(2,4,4-triméthylpentane-2-yl)phénoxy]éthanol (Octoxinol).

De préférence, l'étape d'élimination virale consiste en une nanofiltration qui peut être utilisée pour éliminer les agents infectieux, notamment les virus et les ATNC. Dans le cas des protéines plasmatiques, la nanofiltration se réfère généralement à la filtration du concentré de protéines d'intérêt à travers un filtre avec une taille de pores inférieure à 80 nm. Les filtres disponibles sont par exemple les filtres Planova BioEx, Planova® 75N, Planova® 35N, Planova® 20N ou Planova® 15N (Asahi corporation), Pegasus SV4, Ultipor DV 50 or DV 20 (Pall corporation), Virosart CPV, Virosart HC ou Virosart HF (Sartorius), Viresolve® NFR, Pro ou NFP (Millipore). La nanofiltration peut être avantageusement effectuée sur un filtre unique ou sur plusieurs filtres en série de porosité identique ou décroissante.

L'élimination des agents infectieux peut également être réalisée au moyen d'une filtration en profondeur. Les filtres disponibles sont par exemple des filtres composés de cellulose régénérée, dans lesquels des adjuvants de filtration peuvent avoir été additionnés (tels que la cellite, la perlite ou des terres de Kieselguhr) commercialisés par Cuno (filtres Zeta+ VR series), Pall-Seitz (P-series Depth Filter) ou Sartorius (Virosart CPV, Sartoclear P depth filters).

Après purification et au moins une étape d'élimination ou d'inactivation des agents infectieux, la composition selon l'invention est soumise directement aux étapes de mise en forme pharmaceutique sous forme liquide : formulation, filtration stérilisante et répartition en contenant (flacon ou autre dispositif de conservation/administration).

De manière particulièrement avantageuse, la composition selon l'invention n'est soumise à aucune étape de lyophilisation, dessiccation, déshydratation ou séchage.

De manière particulièrement avantageuse, la composition selon l'invention est donc sous forme liquide sans avoir subi d'étape de reconstitution d'un lyophilisat.

Les exemples suivants illustrent l'invention sans en limiter la portée.

### Exemples :

### Exemple 1 : Composition de fibrinogène liquide stable

Le fibrinogène est obtenu selon la méthode décrite dans le brevet FR 05 06640.

Le produit final est ensuite dyalisé contre un tampon citrate trisodique dihydraté 8.5 mM : (0.175 mM d'acide citrique + 8.325 mM citrate de sodium) à pH 7.0 ± 0.2 afin d'obtenir un fibrinogène déformulé.

### Exemple 2 : Composition de fibrinogène liquide stable

### Matériel & méthode

### Fraction plasmatique

La matière première de départ est un pool de plasma humain préalablement soumis à une étape de cryo-précipitation puis soumis à une étape de précipitation avec 8% d'éthanol.

La solution de fibrinogène plasmatique pré-purifiée ainsi obtenue est diluée par 2 volumes de tampon d'équilibration du gel avant injection sur la colonne de chromatographie pré-équilibrée en pH et en conductivité.

Caractéristiques de la fraction plasmatique : fibrinogène antigénique ajusté par dilution à 4,7-4,8 g/L.

### Solutions tampon

La composition des solutions tampon utilisées lors des différentes étapes du procédé de chromatographie est résumée dans le tableau 1 ci-dessous.

**Tableau 1 : Solutions tampon pour la chromatographie d'affinité.**

| Phases | Composition | Valeurs cibles |
|---|---|---|
| Equilibration de la colonne et retour à la ligne de base | Citrate tri-sodique 10 mM, | pH ajusté à 7,4 |
| | Arginine HCl 0,1 M | |
| Pré-élution | Citrate tri-sodique 10 mM, | pH ajusté à 7,4 |
| | Chlorure de Sodium 2,0 M | |
| | Arginine HCl 0,1 M | |
| Elution | Citrate tri-sodique 10 mM, | pH ajusté à 7,4 |
| | Propylène glycol 50 % v/v | |
| | Arginine HCl 1,0 M | |

### Gels de chromatographie d'affinité

Pour la chromatographie, on utilise un gel d'affinité CaptureSelect Fibrinogen (Life Technologies ref. 191291050, lots 171013-01 et 171013-05).

### Colonnes

Une colonne de 50 mm de diamètre (Référence XK 50/30 GE Healthcare), volume de gel packé de 67 ml ; pour une hauteur de colonne de 3,4 cm a été utilisée

### Purification par affinité

La solution de fibrinogène ajustée à environ 5 g/L est injectée sans autre ajustement sur la colonne d'affinité CaptureSelect Fibrinogène équilibrée. Une charge d'environ 10 g/L a été appliquée.

L'éluat de chromatographie a ensuite été ultrafiltré et pré-formulé sur membrane de seuil de coupure 100 kDa (référence Pall Oméga OS100C10)

A la fin du procédé, le produit obtenu a une concentration en fibrinogène coagulable de 15,2 g/L et en fibrinogène antigénique de 15,2 mg/ml.

### Exemple 3 : stabilité d'une composition de fibrinogène liquide par test de stabilité accéléré

### Compositions de fibrinogène

La composition obtenue à l'exemple 1 ou à l'exemple 2 est formulée avec différents excipients.

Les formulations sont récapitulées dans le tableau 2 ci-dessous :

| ***Formulation*** | ***Fibrinogène*** | ***Glutamate (mM)*** | ***Arginine (mM)*** | ***Acide aminé (mM)*** | ***Citrate trisodique (mM)*** | ***Surfactant*** | ***pH*** | ***Osmolalité*** |
|---|---|---|---|---|---|---|---|---|
| **F1** | Issu de l'exemple 1 | 50 | 50 | | 8,5 | Polysorbate 80 - 200ppm | 7,0 | |
| **F2** | Issu de l'exemple 1 | 150 | 150 | | 8,5 | Polysorbate 80 - 200ppm | 7,0 | 564 |
| **F3** | Issu de l'exemple 1 | 175 | 175 | | 8,5 | Polysorbate 80 - 200ppm | 7,0 | |
| **F4** | Issu de l'exemple 1 | 200 | 200 | | 8,5 | Polysorbate 80 - 200ppm | 7,0 | |
| **F5** | Issu de l'exemple 1 | 300 | 300 | | 8,5 | Polysorbate 80 - 200ppm | 7,0 | |
| **F6** | Issu de l'exemple 1 | 60 | 150 | | 8,5 | Polysorbate 80 - 200ppm | 7,0 | 403 |
| **F7** | Issu de l'exemple 1 | 70 | 150 | | 8,5 | Polysorbate 80 - 200ppm | 7,0 | 423 |
| **F8** | Issu de l'exemple 1 | 80 | 150 | | 8,5 | Polysorbate 80 - 200ppm | 7,0 | 438 |
| **F9** | Issu de l'exemple 1 | 80 | 135 | | 8,5 | Polysorbate 80 - 200ppm | 7,0 | 414 |
| **F10** | Issu de l'exemple 1 | 80 | 120 | | 8,5 | Polysorbate 80 - 200ppm | 7,0 | 387 |
| **F11** | Issu de l'exemple 1 | 80 | 80 | | 8,5 | Polysorbate 80 - 200ppm | 7,0 | |
| **F12** | Issu de l'exemple 1 | 70 | 70 | | 8,5 | Polysorbate 80 - 200ppm | 7,0 | |
| **F13** | Issu de l'exemple 1 | 60 | 60 | | 8,5 | Polysorbate 80 - 200ppm | 7,0 | |
| **F14** | Issu de l'exemple 1 | 50 | 100 | Proline : 60 Glcyine : 27 | 8,5 | Polysorbate 80 - 200ppm | 7,0 | |
| **F15** | Issu de l'exemple 2 | 50 | 100 | Proline : 60 Glcyine : 27 | 8,5 | | 7,0 | |

### Protocoles d'analyses

Les compositions sont soumises à un stress thermique (heating stress) par chauffage en enceinte thermostatée à 37°C puis sorties de l'enceinte thermostatée pour analyse à T0, T+7jours et T+14jours.

Les différentes analyses effectuées sont les suivantes :
- Inspection visuelle : opalescence et formation de particules visibles sont déterminées par inspection visuelle effectuée avec une mireuse Pharmacopée Européenne
- Turbidité
- Analyse des différentes chaînes du fibrinogène par SDS PAGE
- Analyse DLS
- Fibrinogène antigène et activité fibrinogène coagulable

### Résultats

Les résultats obtenus sont les suivants :

**Tableau 3 : Inspection visuelle et turbidité avant et après stress thermique**

| **Stress** | **Composition** | **Opalescence** | **Particules** | **Turbidité** |
|---|---|---|---|---|
| **T0** | F1 | VSOp | 0 | 0.037 |
| | F2 | VSOp | 1 | 0.029 |
| | F3 | VSOp | 0 | 0.03 |
| | F4 | VSOp | 1 | 0.028 |
| | F5 | VSOp | 0 | 0.027 |
| | F6 | S Op | 0 | 0,024 |
| | F7 | S Op | 0 | 0,024 |
| | F8 | S Op | 0 | 0,024 |
| | F9 | S Op | 0 | 0,025 |
| | F10 | S Op | 0 | 0,024 |
| | F11 | S Op | 0 | 0,036 |
| | F12 | S Op | 0 | 0,033 |
| | F13 | S Op | 0 | 0,034 |
| **Stress thermique 37°C, T+7jours** | F1 | Op | 1 | 0.053 |
| | F2 | VSOp | 1 | 0.035 |
| | F3 | VSOp | 1 | 0.034 |
| | F4 | VSOp | 0 | 0.038 |
| | F5 | VSOp | 0 | 0.038 |
| | F6 | S Op | 1 | 0,027 |
| | F7 | S Op | 1 | 0,024 |
| | F8 | S Op | 1 | 0,025 |
| | F9 | S Op | 2 | 0,026 |
| | F10 | S Op | 1 | 0,025 |
| | F11 | S Op | 0 | 0,04 |
| | F12 | S Op | 0 | 0,051 |
| | F13 | S Op | 1 | 0,051 |
| **Stress thermique 37°C, T+14jours** | F1 | S Op | 4 | 0.054 |
| | F2 | S Op | 1 | 0.035 |
| | F3 | V S Op | 0 | 0.033 |
| | F4 | VSOp | 0 | 0.031 |
| | F5 | VSOp | 0 | 0.031 |
| | F6 | S Op | 1 | 0,047 |
| | F7 | S Op | 0 | 0,045 |
| | F8 | S Op | 1 | 0,043 |
| | F9 | S Op | 1 | 0,042 |
| | F10 | S Op | 1 | 0,046 |
| | F11 | S Op | 1 | 0,034 |
| | F12 | S Op | 1 | 0,035 |
| | F13 | Op | 2 | 0,047 |

| | | | | |
|---|---|---|---|---|
| *Légende:* *Opalescence: Op: Opalescent; S Op: légèrement Opalescent, V S Op: très légèrement Opalescent; V Op: très Opalescent* *Particules: 0: sans particules; 1: peu de particules; 2: particules; 3: nombreuses particules; 4: très nombreuses particules* | | | | |

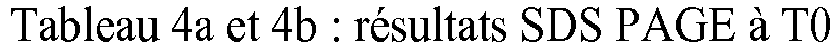

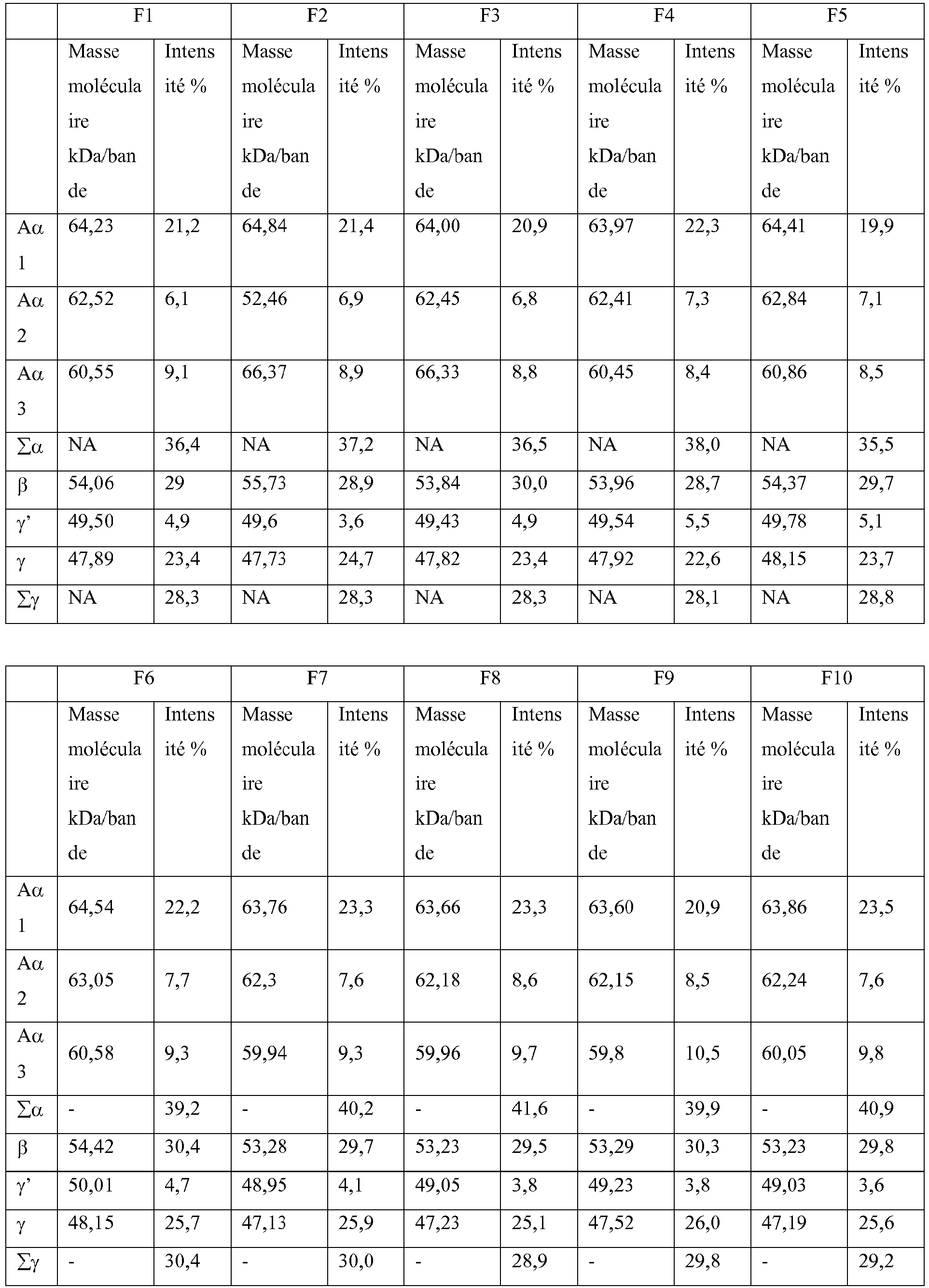

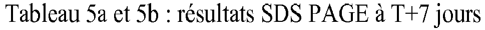

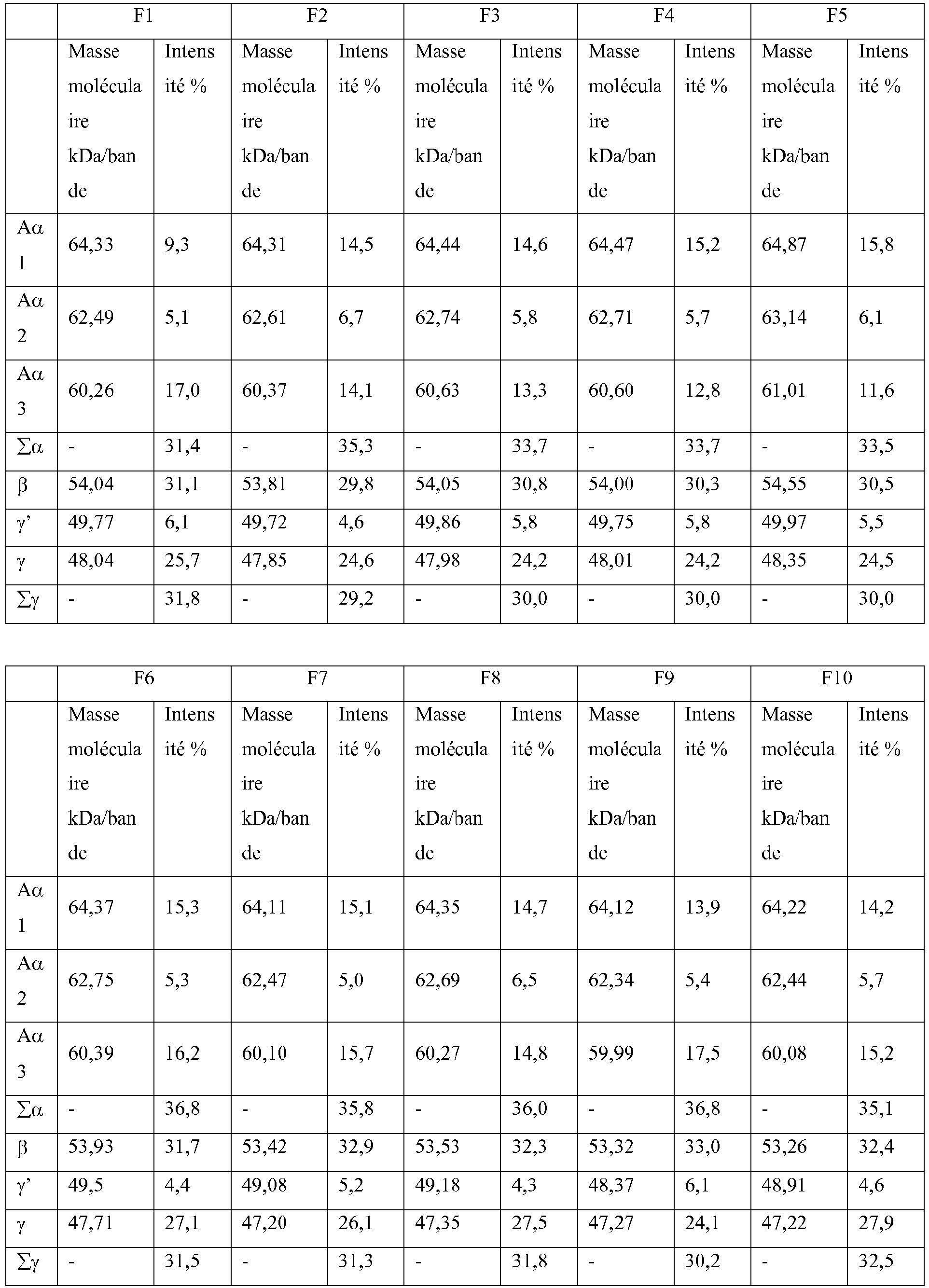

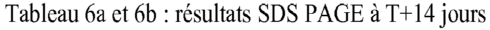

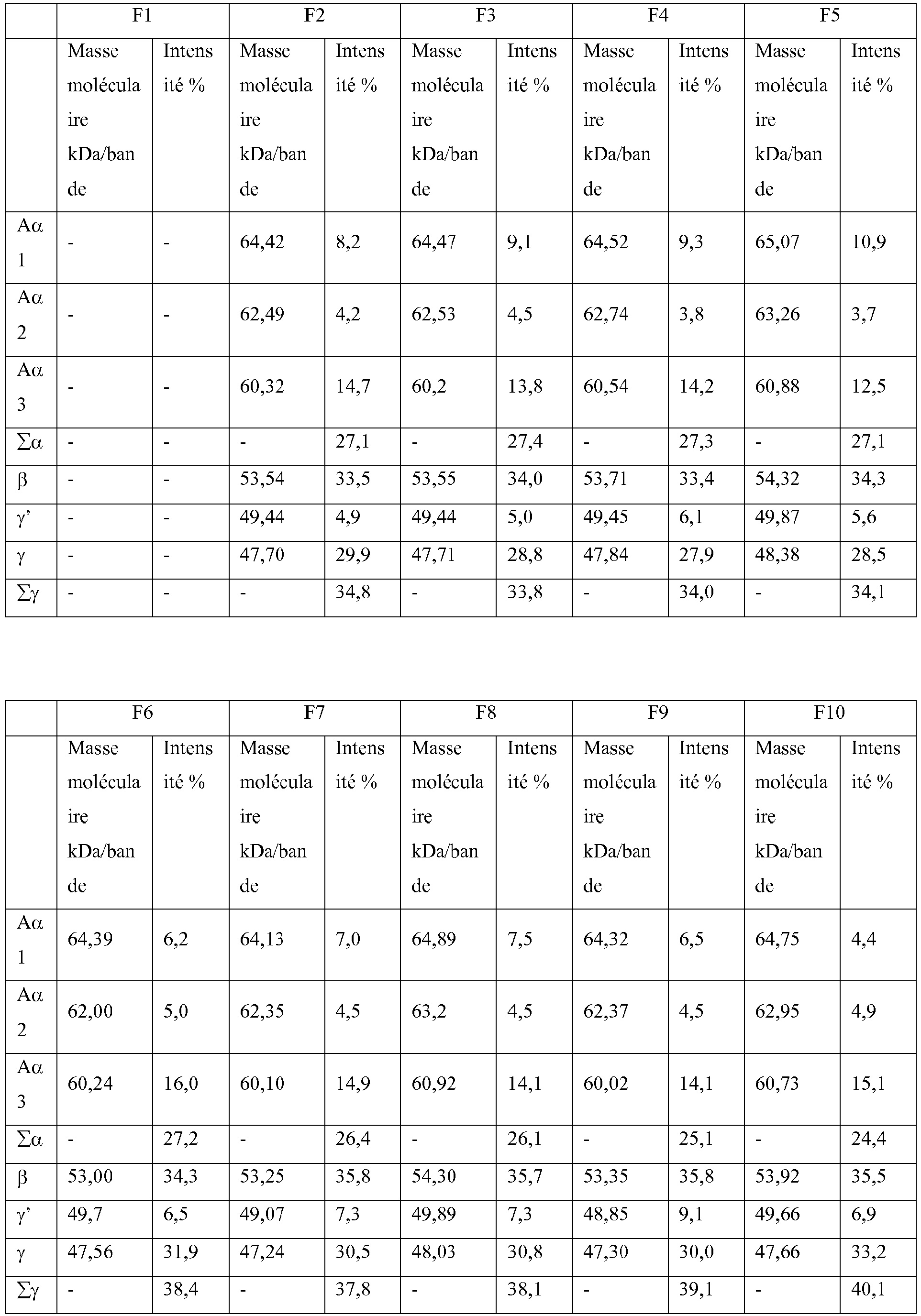

**Tableau 7 : résultats DLS à T0, T+ 7 jours et T+14 jours**

| | T0 | | T + 7 jours | | T + 14 jours | |
|---|---|---|---|---|---|---|
| | Rayon hydrodynamique (nm) | % monomère | Rayon hydrodynamique (nm) | % monomère | Rayon hydrodynamique (nm) | % monomère |
| F1 | 15,1 | 94,8 | 24,1 | 100 | 11,7 | 50 |
| F2 | 14,9 | 98,9 | 17,8 | 98,4 | 16,7 | 92,2 |
| F3 | 14,6 | 96,8 | 13,6 | 72,3 | 16,5 | 92,6 |
| F4 | 14,9 | 91,7 | 15 | 85,2 | 15 | 85,5 |
| F5 | 15,5 | 95,1 | 15,4 | 71 | 17,7 | 97,7 |
| F6 | 14,5 | 98,1 | 13,4 | 69,4 | 14,5 | 55,1 |
| F7 | 14,4 | 98,1 | 14,3 | 77,4 | 13,2 | 53,6 |
| F8 | 14,2 | 95,9 | 15,4 | 85,1 | 13,7 | 57,5 |
| F9 | 14,7 | 97,5 | 15,3 | 82,1 | 14,4 | 63,6 |
| F10 | 14,5 | 94,1 | 16,2 | 89,6 | 15,5 | 69,8 |
| F11 | 15,5 | 97,2 | 15,1 | 64,8 | 12,1 | 57,6 |
| F12 | 15,0 | 95,1 | 15,2 | 64,4 | 11,7 | 54,2 |
| F13 | 16,3 | 100 | 15,4 | 61,6 | 15,3 | 50,24 |

**Tableau 8 : résultats en fibrinogène antigène et en activité fibrinogène coagulable à T0, T+ 7 jours et T+14 jours**

| Activité | F2 | | | F10 | | | F8 | | |
|---|---|---|---|---|---|---|---|---|---|
| | T0 | T7j | T14j | T0 | T7j | T14j | T0 | T7j | T14j |
| Fibrinogène antigène | 15,2 | 15,8 | 15,3 | 15,5 | 15,4 | 15,7 | 15,2 | 15,4 | 15,1 |
| Fibrinogène coagulable | 12,3 | 9,2 | 5,1 | 13,6 | 8,3 | 2,5 | 13,2 | 7,8 | 2,6 |

Les résultats montrent que les formulations F1-F13 à pH 7,0 comprenant entre 10 et 300mM d'arginine et de glutamate, et notamment en quantités équimolaires, sont stables.

### Exemple 4 : stabilité d'une composition de fibrinogène liquide par test de stabilité long terme

### Composition de fibrinogène

Les compositions sont les mêmes que celles de l'exemple 3.

### Stabilité de la composition de fibrinogène

La composition est mise en stabilité à 5°C sous air.

Des échantillons sont prélevés à T0, T+1 mois, T+2 mois, T+3 mois et T+6mois pour analyses.

**Tableaux 9 et 10 : comparaison des formulations F14 et F15 montrant l'effet du tensioactif**

| **F14** | | | **T0** | | **T1M** | | **T2M** | **T3M** | **T6M** | |
|---|---|---|---|---|---|---|---|---|---|---|
| Aspect visuel | | | particle free, very slightly opalescent | | particle free, slightly opalescent | | particle free, very slightly opalescent* | particle free, very slightly opalescent | particle free, very slightly opalescent | |
| DLS | Rayon hydrodynamiqu e des h monomères à 90° (nm) | | 15.2 | | 16.8 | | 15.7 | 16.8 | 17.2 | |
| | monomères à 90° (%) | | 100 | | 100 | | 94.4 | 98.9 | 100 | |
| Turbidité (DO 400nm) | | | 0.036 | | 0.036 | | 0.034 | 0.036 | 0.025 | |
| pH | | | 6.83 | | | | | | 6.8 | |
| Osmolalité | mOsmol/kg | | 353 | | | | | | 394 | |
| fibrinogène Coagulable | | | 14.2 | | 14.0 | | 14.00 | 13.3 | 10.6 | |
| Fibrinogène antigène | | | 15.8 | | 15.4 | | 14.7 | 15.1 | 13.2 | |
| Ratio fibri coag/fibri Ag | | | 0.9 | | 0.91 | | 0.95 | 0.88 | 0.8 | |
| SDS PAGE | Aα1 (64 kDa) | | 21 | | 16.2 | | 11.3 | 10.6 | 3.5 | |
| | Aα2 (62 kDa) | | 8.1 | | 6.8 | | 3.2 | 2.7 | 3.7 | |
| | Aα3 (60 kDa) | | 8.3 | | 15.3 | | 17.2 | 22.4 | 23.5 | |
| | ∑Aαₙ | | 37.4 | | 38.3 | | 31.7 | 35.7 | 30.7 | |
| | Bβ (54 kDa) | | 28.5 | | 28.1 | | 25.7 | 29.1 | 30.5 | |
| | γ' (50 kDa) | | 4.6 | | 4.7 | | 5.7 | 6.9 | 5.6 | |
| | γ (48 kDa) | | 24.6 | | 24.5 | | 22.5 | 23.2 | 28.2 | |
| | ∑γ | | 29.2 | | 29.2 | | 28.2 | 30.1 | 33.8 | |
| HPSEC | Hauts poids moléculaires | | 1.5 | | 2.7 | | 4.7 | 4.6 | 2.5 | |
| | Monomères | | 98.5 | | 97.3 | | 92.3 | 90.6 | 94.3 | |
| | Faibles poids moléculaire | | NA | | NA | | 3 | 4.8 | 3.2 | |
| | | | | | | | | | | |
| **F15** | | | | **T0** | | **T1M** | | **T3M** | | **T6M** |
| Aspect visuel | | | | particle free, very slightly opalescent | | Few particles, slightly opalescent | | Few particles, slightly opalescent | | Few particles, slightly opalescent |
| DLS | | Rayon hydrodynamique des h monomères à 90° (nm) | | 13,6 | | 13,9 | | 13,7 | | 13,3 |
| | | monomères à 90° (%) | | 72 | | 69,2 | | 70,1 | | 63 |
| Turbidité (DO 400nm) | | | | | 0,031 | | 0,039 | | 0,035 | 0,025 |
| pH | | | | | 6,9 | | ND | | ND | 7 |
| Osmolalité | | | mOsmol/kg | | 400 | | ND | | ND | 402 |
| fibrinogène Coagulable | | | | | 13,7 | | ND | | 12,9 | 12 |
| Fibrinogène antigène | | | | | 13,5 | | ND | | 13,9 | 12,3 |
| Ratio fibri coag/fibri Ag | | | | | 1 | | ND | | 0,93 | 0,96 |
| SDS PAGE | | | Aα1 (64 kDa) | | 17,4 | | 16,7 | | 11,8 | 7,2 |
| | | | Aα2 (62 kDa) | | 7,8 | | 7,7 | | 6,8 | 3 |
| | | | Aα3 (60 kDa) | | 7 | | 7,3 | | 12,8 | 16,5 |
| | | | ∑Aαₙ | | 32,2 | | 31,7 | | 31,4 | 26,7 |
| | | | Bβ (54 kDa) | | 31,8 | | 32,1 | | 33,6 | 34,3 |
| | | | γ' (50 kDa) | | 3,9 | | 5,2 | | 6,3 | 5,1 |
| | | | γ (48 kDa) | | 28,2 | | 27,3 | | 25,2 | 30,8 |
| | | | ∑γ | | 32,1 | | 32,5 | | 31,5 | 35,9 |
| HPSEC | | | Hauts poids moléculaires | | ND | | ND | | 3,1 | 3,6 |
| | | | Monomères | | ND | | ND | | 93,3 | 93,7 |
| | | | Faibles poids moléculaire | | ND | | ND | | 3,6 | 2,7 |

### Exemple 5 : stabilité d'une composition de fibrinogène liquide par test de stabilité accéléré

### Compositions de fibrinogène

### La composition obtenue à l'exemple 1 est formulée avec différents excipients.

Les formulations sont récapitulées dans le tableau 11 ci-dessous :

| ***Formulation*** | ***Fibrinogène*** | ***Glutamate (mM)*** | ***Arginine (mM)*** | ***Acide aminé (mM)*** | ***Citrate trisodique (mM)*** | ***Surfactant*** | ***Autre*** | ***pH*** | ***Osmolalité*** |
|---|---|---|---|---|---|---|---|---|---|
| **F16** | 15 g/L , issu de l'exemple 1 | 80 | 150 | | 8,5 | | | 6,0 | 432 |
| **F17** | | 80 | 150 | | 8,5 | | | 7,0 | 434 |
| **F18** | | 50 | 100 | | 8,5 | | | 6,0 | 295 |
| **F19** | | 50 | 200 | | 8,5 | | | 6,0 | 457 |
| **F20** | | 100 | 100 | | 8,5 | | | 6,0 | 385 |
| **F21** | | 100 | 200 | | 8,5 | | | 6,0 | 544 |
| **F22** | | 100 | 200 | Sérine 100 | 8,5 | Polysorbate 80 200ppm | | 6,0 | 518 |
| **F23** | | 100 | 200 | Sérine 100 | 8,5 | Polysorbate 80 200ppm | | 7,0 | 530 |
| **F24** | | 100 | 200 | | 8,5 | Polysorbate 80 200ppm | Albumine 500ppm | 6,0 | 429 |
| **F25** | | 100 | 200 | Sérine 100 | 8,5 | Polysorbate 80 200ppm | Albumine 500ppm | 6,0 | 514 |
| **F26** | | 100 | 200 | Sérine 100 | 8,5 | Polysorbate 80 200ppm | Albumine 500ppm | 7,0 | 516 |

### Protocoles d'analyses

Les compositions sont soumises à un stress thermique (heating stress) par chauffage en enceinte thermostatée à 37°C puis sorties de l'enceinte thermostatée pour analyse à T0, T+7jours et T+14jours.

Les différentes analyses effectuées sont les suivantes :
- Inspection visuelle : opalescence et formation de particules visibles sont déterminées par inspection visuelle effectuée avec une mireuse Pharmacopée Européenne
- Turbidité
- pH
- Osmolalité
- Analyse des différentes chaînes du fibrinogène par SDS PAGE
- Analyse DLS

### Résultats

Les résultats obtenus sont les suivants :
• Inspection visuelle :

| **Formulation** | | **T0** | **T7j 37°C** | **T14j 37°C** |
|---|---|---|---|---|
| F16 | Particles - Ph.Eur | Very slightly opalescent | Very slightly opalescent | Very slightly opalescent |
| | Opalescence - Ph.Eur | Free of particle | Free of particle | Free of particle |
| F17 | Particles - Ph.Eur | Very slightly opalescent | Very slightly opalescent | Very slightly opalescent |
| | Opalescence - Ph.Eur | Free of particle | Few particles | Free of particle |
| F18 | Particles - Ph.Eur | Very slightly opalescent | Slightly opalescent | Slightly opalescent |
| | Opalescence - Ph.Eur | Free of particle | Free of particle | particles |
| F19 | Particles - Ph.Eur | Very slightly opalescent | Very slightly opalescent | Very slightly opalescent |
| | Opalescence - Ph.Eur | Free of particle | Free of particle | Free of particle |
| F20 | Particles - Ph.Eur | Very slightly opalescent | Very slightly opalescent | Very slightly opalescent |
| | Opalescence - Ph.Eur | Free of particle | Free of particle | Free of particle |
| F21 | Particles - Ph.Eur | Very slightly opalescent | Very slightly opalescent | Very slightly opalescent |
| | Opalescence - Ph.Eur | Free of particle | Few particles | Free of particle |
| F22 | Particles - Ph.Eur | Very slightly opalescent | Very slightly opalescent | Very slightly opalescent |
| | Opalescence - Ph.Eur | Free of particle | Few particles | Free of particle |
| F23 | Particles - Ph.Eur | Very slightly opalescent | Very slightly opalescent | Very slightly opalescent |
| | Opalescence - Ph.Eur | Free of particle | Free of particle | Free of particle |
| F24 | Particles - Ph.Eur | Very slightly opalescent | Very slightly opalescent | Very slightly opalescent |
| | Opalescence - Ph.Eur | Free of particle | Free of particle | Free of particle |
| F25 | Particles - Ph.Eur | Very slightly opalescent | Very slightly opalescent | Very slightly opalescent |
| | Opalescence - Ph.Eur | Free of particle | Free of particle | Free of particle |
| F26 | Particles - Ph.Eur | Very slightly opalescent | Very slightly opalescent | Very slightly opalescent |
| | Opalescence - Ph.Eur | Free of particle | Free of particle | Free of particle |

• Turbidité

| **DO 400 nm** | **T0** | **T7j** | **T14j** |
|---|---|---|---|
| F16 | 0,021 | 0,024 | 0,030 |
| F17 | 0,018 | 0,022 | 0,033 |
| F18 | 0,021 | 0,034 | 0,048 |
| F19 | 0,016 | 0,023 | 0,033 |
| F20 | 0,021 | 0,029 | 0,036 |
| F21 | 0,018 | 0,022 | 0,032 |
| F22 | 0,019 | 0,028 | 0,031 |
| F23 | 0,019 | 0,024 | 0,030 |
| F24 | 0,019 | 0,031 | 0,032 |
| F25 | 0,019 | 0,031 | 0,032 |
| F26 | 0,017 | 0,022 | 0,027 |

• Suivi du pH

| **pH** | **T0** | **T7j** | **T14j** |
|---|---|---|---|
| F16 | 6,0 | 6,0 | 6,1 |
| F17 | 7,0 | 7,0 | 7,1 |
| F18 | 6,1 | 6,1 | 6,1 |
| F19 | 6,0 | 6,1 | 6,1 |
| F20 | 6,1 | 6,1 | 6,1 |
| F21 | 6,0 | 6,0 | 6,0 |
| F22 | 6,1 | 6,1 | 6,1 |
| F23 | 7,0 | 6,9 | 7,0 |
| F24 | 6,0 | 6,0 | 6,1 |
| F25 | 6,0 | 6,0 | 6,0 |
| F26 | 6,9 | 6,9 | 6,9 |

• Osmolalité

| **Formulation** | **T0** | **T7j** | **T14j** |
|---|---|---|---|
| F16 | 432 | 433 | 431 |
| F17 | 434 | 438 | 435 |
| F18 | 295 | 295 | 294 |
| F19 | 457 | 465 | 462 |
| F20 | 385 | 386 | 385 |
| F21 | 544 | 546 | 545 |
| F22 | 518 | 516 | 515 |
| F23 | 530 | 534 | 526 |
| F24 | 429 | 431 | 429 |
| F25 | 514 | 514 | 516 |
| F26 | 516 | 513 | 512 |

• Analyse DLS

| **Formulation** | | **T0** | **T7j** | **T14J** |
|---|---|---|---|---|
| F16 | Rh (nm) | 14,0 | 15,6 | 14,9 |
| | %monomère | 100 | 96,0 | 80,2 |
| | ITD (u.a.) | 19,42 | 23,03 | 25,13 |
| F17 | Rh (nm) | 13,8 | 13,9 | 14,6 |
| | %monomère | 100 | 93,3 | 85,2 |
| | ITD (u.a.) | 19,10 | 21,23 | 23,34 |
| F18 | Rh (nm) | 14,5 | 13,2 | 14,0 |
| | %monomère | 100 | 64,3 | 58,8 |
| | ITD (u.a.) | 24,26 | 32,43 | 38,25 |
| F19 | Rh (nm) | 13,8 | 12,8 | 13,3 |
| | %monomère | 100 | 71,3 | 59,8 |
| | ITD (u.a.) | 20,02 | 24,59 | 29,75 |
| F20 | Rh (nm) | 14,8 | 13,9 | 14,0 |
| | %monomère | 100 | 80,0 | 71,6 |
| | ITD (u.a.) | 23,08 | 27,24 | 29,21 |
| F21 | Rh (nm) | 13,9 | 15,6 | 13,2 |
| | %monomère | 100 | 97,7 | 73,3 |
| | ITD (u.a.) | 19,52 | 22,00 | 24,16 |
| F22 | Rh (nm) | 14,4 | 13,7 | 13,7 |
| | %monomère | 100 | 74,5 | 64,2 |
| | ITD (u.a.) | 19,22 | 25,03 | 28,17 |
| F23 | Rh (nm) | 13,6 | 13,9 | 14,6 |
| | %monomère | 97,9 | 87,3 | 79,9 |
| | ITD (u.a.) | 18,93 | 21,85 | 24,02 |
| F24 | Rh (nm) | 13,6 | 12,8 | 12,0 |
| | %monomère | 98,9 | 74,8 | 56,5 |
| | ITD (u.a.) | 19,63 | 23,85 | 27,65 |
| F25 | Rh (nm) | 14,0 | 13,9 | 13,1 |
| | %monomère | 100 | 83,1 | 66,5 |
| | ITD (u.a.) | 19,25 | 22,98 | 27,06 |
| F26 | Rh (nm) | 13,9 | 15,1 | 14,1 |
| | %monomère | 100 | 100,0 | 83,8 |
| | ITD (u.a.) | 19,09 | 20,61 | 22,31 |

A T0

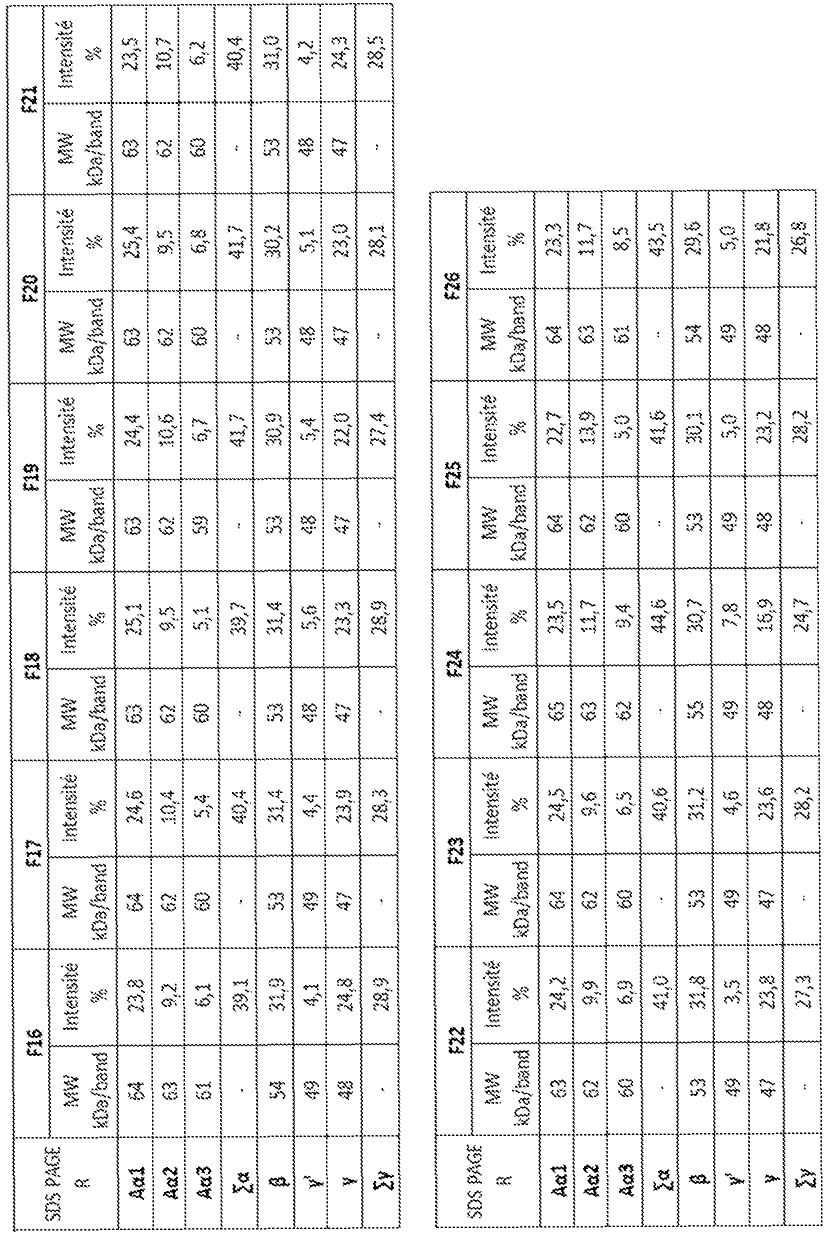

A T+7j

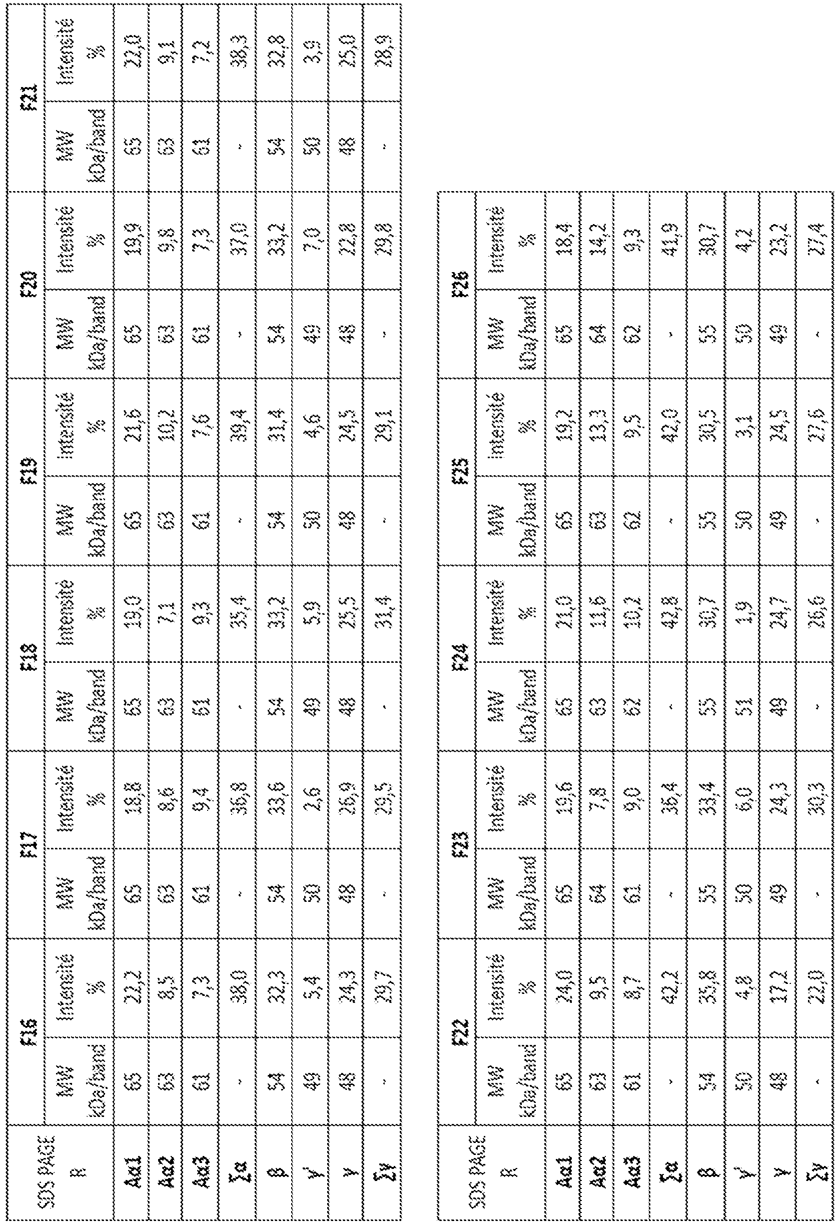

A T+14J

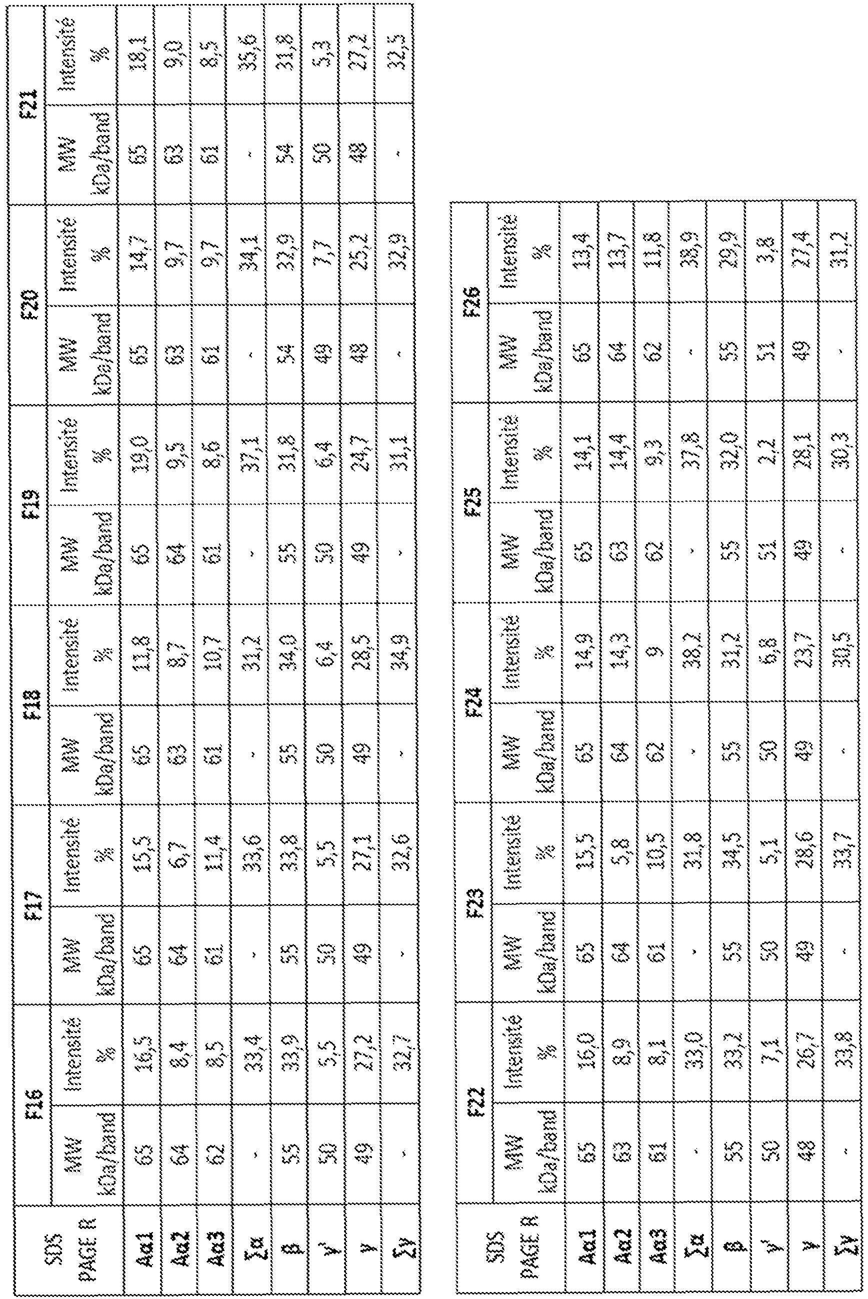

Les résultats montrent que les formulations F16-F26 à pH 6,0-8,0 comprenant entre 50 et 200 mM d'arginine et de glutamate sont stables.

### Exemple 6 : stabilité d'une composition de fibrinogène liquide par test de stabilité long terme

### Compositions de fibrinogène

La composition obtenue à l'exemple 2 est formulée avec différents excipients.

Les formulations sont récapitulées dans le tableau 11 ci-dessous :

| ***Formulation*** | ***Fibrittogène*** | ***Glutamate (mM)*** | ***Arginine (mM)*** | ***Acide aminé (mM)*** | ***Citrate trisodique (mM)*** | ***Surfactant*** | ***Autre*** | ***pH*** | ***Osmolalité*** |
|---|---|---|---|---|---|---|---|---|---|
| **F27** | 15 g/L, issu de l'exemple 2 | 80 | 150 | Sérine 100 | 8,5 | Polysorbate 80 200 ppm | | 6,0 | 533 |
| **F28** | | 80 | 150 | Sérine 100 | 8,5 | Polysorbate 80 200 ppm | | 7,0 | 529 |

### Protocoles d'analyses

Les compositions sont conservées en enceinte thermostatée à 5°C, 25°C et 37°C puis sorties de l'enceinte thermostatée pour analyse à T0, T+14jours et T+1mois.

Les différentes analyses effectuées sont les suivantes :
- Inspection visuelle : opalescence et formation de particules visibles sont déterminées par inspection visuelle effectuée avec une mireuse Pharmacopée Européenne
- Turbidité
- pH
- Osmolalité
- Analyse DLS
- Fibrinogène coagulable et fibrinogène antigène

Analyse des différentes chaînes du fibrinogène par SDS PAGE

### Résultats

Les résultats obtenus sont les suivants :
• Inspection visuelle :

| **F27** | | **T0** | | **T14j** | | **T1M** | |
|---|---|---|---|---|---|---|---|
| | | Particule Ph. Eur. | Opalescence Ph .Eur. | Particule Ph. Eur. | Opalescence Ph .Eur. | Particule Ph. Eur. | Opalescence Ph .Eur. |
| **Inspection visuelle** | **5°C** | 0 | S op | | | | |
| | **25°C** | | | | | 0 | V S op |
| | **37°C** | | | 0 | S op | 0 | S op |
| | | | | | | | |

| **F28** | | **T0** | | **T14j** | | **T1M** | |
|---|---|---|---|---|---|---|---|
| | | Particule Ph. Eur. | Opalescence Ph .Eur. | Particule Ph. Eur. | Opalescence Ph .Eur. | Particule Ph. Eur. | Opalescence Ph .Eur. |
| **Inspection visuelle** | **5°C** | 0 | S op | | | | |
| | **25°C** | | | | | 0 | V S op |
| | **37°C** | | | 0 | S op | 0 | S op |

• Turbidité

| **F27** | | **T0** | **T14d** | **T1M** |
|---|---|---|---|---|
| **Turbidité** | **5°C** | 0,008 | | |
| | **25°C** | | | 0,026 |
| | **37°C** | | 0,046 | 0,055 |
| | | | | |

| **F28** | | **T0** | **T14d** | **T1M** |
|---|---|---|---|---|
| **Turbidité** | **5°C** | 0,011 | | |
| | **25°C** | | | 0,027 |
| | **37°C** | | 0,035 | 0,043 |

• pH et osmolalité

| **F27** | | **T0** | **T14d** | **T1M** |
|---|---|---|---|---|
| **pH** | **5°C** | 6,1 | | |
| | **25°C** | | | 6,1 |
| | **37°C** | | 6,1 | 6,1 |
| **osmolalité** | **5°C** | 533 | | |
| | **25°C** | | | 534 |
| | **37°C** | | 534 | 536 |
| | | | | |

| **F28** | | **T0** | **T14d** | **T1M** |
|---|---|---|---|---|
| **pH** | **5°C** | 6,9 | | |
| | **25°C** | | | 6,9 |
| | **37°C** | | 6,9 | 6,9 |
| **osmolalité** | **5°C** | 529 | | |
| | **25°C** | | | 534 |
| | **37°C** | | 534 | 533 |

• Analyse DLS

| **F27** | | **T0** | | **T14j** | | **T1M** | |
|---|---|---|---|---|---|---|---|
| | | Monomère à 90° (%) | ITD à 90° (u.a.) | Monomère à 90° (%) | ITD à 90° (u.a.) | Monomère à 90° (%) | ITD à 90° (u.a.) |
| **DLS** | **5°C** | 100 | 25,43 | | | | |
| | **25°C** | | | | | 96,2 | 28,89 |
| | **37°C** | | | 38,9 | 56,27 | 30,7 | 71,90 |
| | | | | | | | |

| **F28** | | **T0** | | **T14j** | | **T1M** | |
|---|---|---|---|---|---|---|---|
| | | Monomère à 90° (%) | ITD à 90° (u.a.) | Monomère à 90° (%) | ITD à 90° (u.a.) | Monomère à t 90° (%) | ITD à 90° (u.a.) |
| **DLS** | **5°C** | 98,4 | 25,15 | | | | |
| | **25°C** | | | | | 90,4 | 28,24 |
| | **37°C** | | | 46,9 | 48,07 | 37,5 | 57,38 |

• Fibrinogène coagulable et fibrinogène antigène

| **Stabilité à+5°C** | | **F27** | **F28** |
|---|---|---|---|
| **T0** | Fg activité (g/L) | 15,7 | 15,9 |
| | Fg antigène | 15,7 | 15,2 |
| | *Ratio* | *1,0* | *1,0* |
| | | | |

| **Stabilité** à **+25°C** | | **F27** | **F28** |
|---|---|---|---|
| **T0** | Fg activité (g/L) | 15,7 | 15,9 |
| | Fg antigène | 15,7 | 15,2 |
| | *Ratio* | *1,0* | *1,0* |
| **T1M** | Fg activité | 14,3 | 12,7 |
| | (g/L) | | |
| | Fg antigène | 16,0 | 15,2 |
| | *Ratio* | *0,9* | *0,8* |

• Analyse des différentes chaînes du fibrinogène par SDS PAGE en conditions réductrices

| **F27** | | | | |
|---|---|---|---|---|
| **mW (Kda)** | **T0** | **T14j 37°C** | **T1M 25°C** | **T1M 37°C** |
| **219** | 2,1 | 2,4 | 2,6 | 2,2 |
| **153** | 0,9 | 1,1 | 0,7 | 0,8 |
| **101** | 1,7 | 1,4 | 1,3 | 1,5 |
| **64 Aα1** | 23,2 | 16,6 | 20,0 | 16,0 |
| **62 Aα2** | 9,7 | 9 | 7,5 | 7,5 |
| **60 Aα3** | 2 | 3,6 | 3,3 | 4,4 |
| **Σ64/62/60** | **34,9** | **29,2** | **30,8** | **27,9** |
| **54 Bβ** | **31,4** | **34,1** | **33,3** | **34,2** |
| **50 γ'** | 2,9 | 3,1 | 2,8 | 2,7 |
| **48γ** | 25,7 | 27,6 | 28,0 | 30,0 |
| **Σ50/48** | **28,6** | **30,7** | **30,8** | **32,7** |
| **34** | 0,3 | 1 | 0,4 | 0,7 |
| **29** | nd | nd | nd | nd |
| | | | | |

| **F28** | | | | |
|---|---|---|---|---|
| **mW (Kda)** | **T0** | **T14j 37°C** | **T1M 25°C** | **T1M 37°C** |
| **219** | 2,8 | 2,3 | 2,4 | 2,5 |
| **153** | 0,8 | 0,8 | 1,0 | 0,9 |
| **101** | 1,8 | 1,8 | 1,7 | 2,1 |
| **64 Aα1** | 23,7 | 16 | 12,5 | 9,4 |
| **62 Aα2** | 8,2 | 7,8 | 8,1 | 7,8 |
| **60 Aα3** | 2,0 | 4,0 | 3,0 | 4,8 |
| **Σ64/62/60** | **33,9** | **27,8** | **23,6** | **22,0** |
| **54 Bβ** | **31,4** | **33,9** | **35,0** | **35,1** |
| **50 γ'** | 2,3 | 2,7 | 3,2 | 6,6 |
| **48γ** | 26,6 | 29,6 | 30,5 | 29,8 |
| **Σ50/48** | **28,9** | **32,3** | **33,7** | **36,4** |
| **34** | 0,6 | 1,2 | 1,0 | 0,8 |
| **29** | nd | nd | 1,5 | 1,2 |

Les résultats montrent que les formulations F27-F28 à pH 6,0-7,0 comprenant 80mM de glutamate et 150mM d'arginine, en présence de surfactant, de tampon et d'un acide aminé supplémentaire, sont stables.

## Revendications

1. Composition pharmaceutique liquide comprenant du fibrinogène humain **caractérisée en ce qu'**elle comprend :
- entre 10 et 300 mM d'arginine
- entre 10 et 300 mM de glutamate
le pH de la composition étant compris entre 6 et 8.

2. Composition pharmaceutique selon la revendication 1, **caractérisée en ce que** la concentration en arginine et la concentration en glutamate est comprise entre 10 et 300 mM, préférentiellement entre 20 et 200 mM, plus préférentiellement entre 30 et 100 mM, encore plus préférentiellement entre 50 et 80 mM.

3. Composition pharmaceutique selon la revendication 1, **caractérisée en ce que** la concentration en arginine et la concentration en glutamate est comprise entre 10 et 80 mM, préférentiellement comprise entre 20 et 70 mM, plus préférentiellement comprise entre 30 et 60 mM.

4. Composition pharmaceutique selon l'une quelconque des revendications précédentes, **caractérisée en ce que** la concentration en glutamate est inférieure à 80 mM, préférentiellement inférieure à 70 mM, plus préférentiellement inférieure à 60 mM, encore plus préférentiellement inférieure à 50 mM.

5. Composition pharmaceutique selon l'une quelconque des revendications précédentes, **caractérisée en ce que** la concentration en glutamate est comprise entre 10 et 80 mM, préférentiellement comprise entre 20 et 70 mM, plus préférentiellement comprise entre 30 et 60 mM.

6. Composition pharmaceutique selon l'une quelconque des revendications précédentes, **caractérisée en ce que** la concentration en arginine est comprise entre 10 et 300 mM, préférentiellement comprise entre 20 et 250 mM, plus préférentiellement comprise entre 50 et 250 mM.

7. Composition pharmaceutique selon l'une quelconque des revendications précédentes, **caractérisée en ce que** l'arginine et le glutamate sont présents en quantités équimolaires à un ratio compris entre 0,8 et 1,2, préférentiellement entre 0,9 et 1,1, encore plus préférentiellement environ égal à 1,0.

8. Composition pharmaceutique selon l'une quelconque des revendications précédentes, **caractérisée en ce que** la concentration en fibrinogène est comprise entre 10 et 30 g/L, préférentiellement entre 15 et 25 g/L, encore plus préférentiellement entre 15 et 20 g/L.

9. Composition pharmaceutique selon l'une quelconque des revendications précédentes, **caractérisée en ce qu'**elle comprend également au moins un acide aminé choisi parmi l'alanine, l'asparagine, l'aspartate, la cystéine, la glutamine, la glycine, l'histidine, l'isoleucine, la leucine, la lysine, la méthionine, la phénylalanine, la proline, la sérine, la thréonine, le tryptophane, la tyrosine, la valine, ou une combinaison de ceux-ci.

10. Composition pharmaceutique selon l'une quelconque des revendications précédentes, **caractérisée en ce qu'**elle comprend également au moins un acide aminé hydrophobe choisi parmi la leucine, l'alanine, la phénylalanine, le tryptophane, la valine, la méthionine, l'isoleucine, la proline, la cystéine et/ou la glycine ou une combinaison de ceux-ci.

11. Composition pharmaceutique selon la revendication 10, **caractérisée en ce que** l'acide aminé est choisi parmi la proline et/ou l'isoleucine et/ou la sérine.

12. Composition pharmaceutique selon l'une quelconque des revendications 9 à 11, **caractérisée en ce que** la concentration en acide aminé est comprise entre 1 et 300 mM, préférentiellement entre 10 et 200 mM, plus préférentiellement entre 20 et 100 mM.

13. Composition pharmaceutique selon l'une quelconque des revendications précédentes, **caractérisée en ce qu'**elle comprend également un surfactant, préférentiellement du polysorbate 80 ou du polysorbate 20 ou du Pluronic®F68.

14. Composition pharmaceutique selon la revendication 13, **caractérisée en ce que** la concentration en surfactant est comprise entre 1 et 500 ppm, préférentiellement entre 50 et 400 ppm, plus préférentiellement entre 150 et 250 ppm.

15. Composition pharmaceutique selon l'une quelconque des revendications précédentes, **caractérisée en ce qu'**elle comprend également du tampon.

16. Composition pharmaceutique la revendication 15, **caractérisée** en ce le tampon consiste en du citrate trisodique.

17. Composition pharmaceutique selon la revendication 16, **caractérisée en ce que** la concentration en citrate trisodique est comprise entre 1 et 15 mM, préférentiellement entre 7 et 10 mM

18. Composition pharmaceutique selon l'une quelconque des revendications précédentes, **caractérisée en ce que** le pH est compris entre 6 et 7,5, préférentiellement entre 6 et 7.

19. Composition pharmaceutique selon l'une quelconque des revendications précédentes, **caractérisée en ce qu'**elle est dépourvue de chlorure de sodium et/ou d'albumine.

20. Composition pharmaceutique selon l'une quelconque des revendications précédentes, **caractérisée en ce qu'**elle présente une osmolalité comprise entre 250 et 650 mOsm/kg, préférentiellement entre 300 et 550 mOsm/kg.

21. Composition pharmaceutique liquide selon l'une quelconque des revendications précédentes, **caractérisée en ce qu'**elle comprend :
- 10-30 g/L de fibrinogène
- 10-300 mM d'arginine
- 10-300 mM de glutamate
- éventuellement 1-15 mM de tampon, en particulier de citrate trisodique
- éventuellement 1-300 mM d'un autre acide aminé, en particulier 1-300 mM de proline et/ou 1-300 mM d'isoleucine et/ou 1-300 mM de sérine
- éventuellement 1-500 ppm de surfactant, préférentiellement 20-400 ppm, de manière encore préférée 150-250 ppm
- éventuellement 5-1000 ppm d'albumine humaine
le pH de la composition étant de 6,0-8,0.

22. Composition pharmaceutique liquide selon l'une quelconque des revendications précédentes, **caractérisée en ce qu'**elle comprend :
- 10-30 g/L de fibrinogène
- 20-200 mM d'arginine
- 20-200 mM de glutamate
- éventuellement 7-10 mM de tampon, en particulier de citrate trisodique
- éventuellement 1-100 mM d'un autre acide aminé, en particulier 1-100 mM de proline et/ou 1-100 mM d'isoleucine et/ou 1-100 mM de sérine
- éventuellement 20-400 ppm de surfactant
- éventuellement 5-500 ppm d'albumine humaine
le pH de la composition étant de 6,0-8,0.

23. Composition pharmaceutique liquide selon l'une quelconque des revendications précédentes, **caractérisée en ce qu'**elle comprend :
- 15-25 g/L de fibrinogène
- 150 mM d'arginine
- 80 mM de glutamate
- 8,5 mM de citrate trisodique
- 200 ppm de surfactant, préférentiellement de polysorbate80, de polysorbate20 ou de Pluronic®F68
- éventuellement 1-100 mM d'un autre acide aminé, en particulier 1-100 mM de proline et/ou 1-100 mM d'isoleucine et/ou 1-100 mM de sérine
- éventuellement 5-1000 ppm d'albumine humaine
le pH de la composition étant de 6,0-7,0.

24. Composition pharmaceutique selon l'une quelconque des revendications précédentes, **caractérisée en ce que** le fibrinogène est obtenu par fractionnement de plasma sanguin.

## Patentansprüche

1. Flüssige pharmazeutische Zusammensetzung, umfassend humanes Fibrinogen, **dadurch gekennzeichnet, dass** sie umfasst:
- zwischen 10 und 300 mM Arginin,
- zwischen 10 und 300 mM Glutamat,
wobei der pH-Wert der Zusammensetzung zwischen 6 und 8 liegt.

2. Pharmazeutische Zusammensetzung nach Anspruch 1, **dadurch gekennzeichnet, dass** die Argininkonzentration und die Glutamatkonzentration zwischen 10 und 300 mM liegt, vorzugsweise zwischen 20 und 200 mM, weiter bevorzugt zwischen 30 und 100 mM, noch bevorzugter zwischen 50 und 80 mM.

3. Pharmazeutische Zusammensetzung nach Anspruch 1, **dadurch gekennzeichnet, dass** die Argininkonzentration und die Glutamatkonzentration zwischen 10 und 80 mM liegt, vorzugsweise zwischen 20 und 70 mM, weiter bevorzugt zwischen 30 und 60 mM.

4. Pharmazeutische Zusammensetzung nach einem der vorhergehenden Ansprüche, **dadurch gekennzeichnet, dass** die Glutamatkonzentration weniger als 80 mM beträgt, vorzugsweise weniger als 70 mM, weiter bevorzugt weniger als 60 mM, noch bevorzugter weniger als 50 mM.

5. Pharmazeutische Zusammensetzung nach einem der vorhergehenden Ansprüche, **dadurch gekennzeichnet, dass** die Glutamatkonzentration zwischen 10 und 80 mM liegt, vorzugsweise zwischen 20 und 70 mM, weiter bevorzugt zwischen 30 und 60 mM.

6. Pharmazeutische Zusammensetzung nach einem der vorhergehenden Ansprüche, **dadurch gekennzeichnet, dass** die Argininkonzentration zwischen 10 und 300 mM liegt, vorzugsweise zwischen 20 und 250 mM, weiter bevorzugt zwischen 50 und 250 mM.

7. Pharmazeutische Zusammensetzung nach einem der vorhergehenden Ansprüche, **dadurch gekennzeichnet, dass** Arginin und Glutamat in äquimolaren Mengen in einem Verhältnis zwischen 0,8 und 1,2 vorliegen, vorzugsweise zwischen 0,9 und 1,1, weiter bevorzugt etwa 1,0.

8. Pharmazeutische Zusammensetzung nach einem der vorhergehenden Ansprüche, **dadurch gekennzeichnet, dass** die Fibrinogenkonzentration zwischen 10 und 30 g/L liegt, vorzugsweise zwischen 15 und 25 g/L, noch weiter bevorzugt zwischen 15 und 20 g/L.

9. Pharmazeutische Zusammensetzung nach einem der vorhergehenden Ansprüche, **dadurch gekennzeichnet, dass** sie außerdem mindestens eine Aminosäure, ausgewählt aus Alanin, Asparagin, Aspartat, Cystein, Glutamin, Glycin, Histidin, Isoleucin, Leucin, Lysin, Methionin, Phenylalanin, Prolin, Serin, Threonin, Tryptophan, Tyrosin, Valin, oder einer Kombination davon, enthält.

10. Pharmazeutische Zusammensetzung nach einem der vorhergehenden Ansprüche, **dadurch gekennzeichnet, dass** sie außerdem mindestens eine hydrophobe Aminosäure, ausgewählt aus Leucin, Alanin, Phenylalanin, Tryptophan, Valin, Methionin, Isoleucin, Prolin, Cystein und/oder Glycin oder einer Kombination davon, enthält.

11. Pharmazeutische Zusammensetzung nach Anspruch 10, **dadurch gekennzeichnet, dass** die Aminosäure ausgewählt ist aus Prolin und/oder Isoleucin und/oder Serin.

12. Pharmazeutische Zusammensetzung nach einem der Ansprüche 9 bis 11, **dadurch gekennzeichnet, dass** die Aminosäurekonzentration zwischen 1 und 300 mM liegt, vorzugsweise zwischen 10 und 200 mM, weiter bevorzugt zwischen 20 und 100 mM.

13. Pharmazeutische Zusammensetzung nach einem der vorhergehenden Ansprüche, **dadurch gekennzeichnet, dass** sie außerdem ein Tensid enthält, vorzugsweise Polysorbat 80 oder Polysorbat 20 oder Pluronic®F68.

14. Pharmazeutische Zusammensetzung nach Anspruch 13, **dadurch gekennzeichnet, dass** die Tensidkonzentration zwischen 1 und 500 ppm liegt, vorzugsweise zwischen 50 und 400 ppm, weiter bevorzugt zwischen 150 und 250 ppm.

15. Pharmazeutische Zusammensetzung nach einem der vorhergehenden Ansprüche, **dadurch gekennzeichnet, dass** sie außerdem Puffer enthält.

16. Pharmazeutische Zusammensetzung Anspruch 15, **dadurch gekennzeichnet, dass** der Puffer aus Trinatriumcitrat besteht.

17. Pharmazeutische Zusammensetzung nach Anspruch 16, **dadurch gekennzeichnet, dass** die Konzentration an Trinatriumcitrat zwischen 1 und 15 mM liegt, vorzugsweise zwischen 7 und 10 mM.

18. Pharmazeutische Zusammensetzung nach einem der vorhergehenden Ansprüche, **dadurch gekennzeichnet, dass** der pH-Wert zwischen 6 und 7,5 liegt, vorzugsweise zwischen 6 und 7.

19. Pharmazeutische Zusammensetzung nach einem der vorhergehenden Ansprüche, **dadurch gekennzeichnet, dass** sie frei von Natriumchlorid und/oder Albumin ist.

20. Pharmazeutische Zusammensetzung nach einem der vorhergehenden Ansprüche, **dadurch gekennzeichnet, dass** sie eine Osmolalität zwischen 250 und 650 mOsm/kg aufweist, vorzugsweise zwischen 300 und 550 mOsm/kg.

21. Flüssige pharmazeutische Zusammensetzung nach einem der vorhergehenden Ansprüche, **dadurch gekennzeichnet, dass** sie umfasst:
- 10-30 g/L Fibrinogen
- 10-300 mM Arginin
- 10-300 mM Glutamat
- gegebenenfalls 1-15 mM Puffer, insbesondere Trinatriumcitrat
- gegebenenfalls 1-300 mM einer anderen Aminosäure, insbesondere 1-300 mM Prolin und/oder 1-300 mM Isoleucin und/oder 1-300 mM Serin
- gegebenenfalls 1-500 ppm Tensid, vorzugsweise 20-400 ppm, weiter bevorzugt 150-250 ppm
- gegebenenfalls 5-1000 ppm humanes Albumin,
wobei der pH-Wert der Zusammensetzung 6,0-8,0 beträgt.

22. Flüssige pharmazeutische Zusammensetzung nach einem der vorhergehenden Ansprüche, **dadurch gekennzeichnet, dass** sie umfasst:
- 10-30 g/L Fibrinogen
- 20-200 mM Arginin
- 20-200 mM Glutamat
- gegebenenfalls 7-10 mM Puffer, insbesondere Trinatriumcitrat
- gegebenenfalls 1-100 mM einer anderen Aminosäure, insbesondere 1-100 mM Prolin und/oder 1-100 mM Isoleucin und/oder 1-100 mM Serin
- gegebenenfalls 20-400 ppm Tensid
- gegebenenfalls 5-500 ppm humanes Albumin,
wobei der pH-Wert der Zusammensetzung 6,0-8,0 beträgt.

23. Flüssige pharmazeutische Zusammensetzung nach einem der vorhergehenden Ansprüche, **dadurch gekennzeichnet, dass** sie umfasst:
- 15-25 g/L Fibrinogen
- 150 mM Arginin
- 80 mM Glutamat
- 8,5 mM Trinatriumcitrat
- 200 ppm Tensid, vorzugsweise Polysorbat80, Polysorbat20 oder Pluronic®F68
- gegebenenfalls 1-100 mM einer anderen Aminosäure, insbesondere 1-100 mM Prolin und/oder 1-100 mM Isoleucin und/oder 1-100 mM Serin
- gegebenenfalls 5-1000 ppm humanes Albumin,
wobei der pH-Wert der Zusammensetzung 6,0-7,0 beträgt.

24. Pharmazeutische Zusammensetzung nach einem der vorhergehenden Ansprüche, **dadurch gekennzeichnet, dass** das Fibrinogen durch Fraktionierung von Blutplasma gewonnen wird.

## Claims

1. Liquid pharmaceutical composition comprising human fibrinogen, **characterized in that** it comprises:
- between 10 and 300 mM of arginine
- between 10 and 300 mM of glutamate,
the pH of the composition being between 6 and 8.

2. Pharmaceutical composition according to Claim 1, **characterized in that** the arginine concentration and the glutamate concentration is between 10 and 300 mM, preferably between 20 and 200 mM, more preferably between 30 and 100 mM, more preferably still between 50 and 80 mM.

3. Pharmaceutical composition according to Claim 1, **characterized in that** the arginine concentration and the glutamate concentration is between 10 and 80 mM, preferably between 20 and 70 mM, more preferably between 30 and 60 mM.

4. Pharmaceutical composition according to any one of the preceding claims, **characterized in that** the glutamate concentration is less than 80 mM, preferably less than 70 mM, more preferably less than 60 mM, more preferably still less than 50 mM.

5. Pharmaceutical composition according to any one of the preceding claims, **characterized in that** the glutamate concentration is between 10 and 80 mM, preferably between 20 and 70 mM, more preferably between 30 and 60 mM.

6. Pharmaceutical composition according to any one of the preceding claims, **characterized in that** the arginine concentration is between 10 and 300 mM, preferably between 20 and 250 mM, more preferably between 50 and 250 mM.

7. Pharmaceutical composition according to any one of the preceding claims, **characterized in that** the arginine and the glutamate are present in equimolar amounts at a ratio of between 0.8 and 1.2, preferably between 0.9 and 1.1, more preferably approximately equal to 1.0.

8. Pharmaceutical composition according to any one of the preceding claims, **characterized in that** the fibrinogen concentration is between 10 and 30 g/L, preferably between 15 and 25 g/L, more preferably still between 15 and 20 g/L.

9. Pharmaceutical composition according to any one of the preceding claims, **characterized in that** it further comprises at least one amino acid selected from alanine, asparagine, aspartate, cysteine, glutamine, glycine, histidine, isoleucine, leucine, lysine, methionine, phenylalanine, proline, serine, threonine, tryptophan, tyrosine, valine, or a combination thereof.

10. Pharmaceutical composition according to any one of the preceding claims, **characterized in that** it further comprises at least one hydrophobic amino acid selected from leucine, alanine, phenylalanine, tryptophan, valine, methionine, isoleucine, proline, cysteine, and/or glycine or a combination thereof.

11. Pharmaceutical composition according to Claim 10, **characterized in that** the amino acid is selected from proline and/or isoleucine and/or serine.

12. Pharmaceutical composition according to any one of Claims 9 to 11, **characterized in that** the amino acid concentration is between 1 and 300 mM, preferably between 10 and 200 mM, more preferably between 20 and 100 mM.

13. Pharmaceutical composition according to any one of the preceding claims, **characterized in that** it further comprises a surfactant, preferably polysorbate 80 or polysorbate 20 or Pluronic®F68.

14. Pharmaceutical composition according to Claim 13, **characterized in that** the surfactant concentration is between 1 and 500 ppm, preferably between 50 and 400 ppm, more preferably between 150 and 250 ppm.

15. Pharmaceutical composition according to any one of the preceding claims, **characterized in that** it further comprises buffer.

16. Pharmaceutical composition according to Claim 15, **characterized in that** the buffer consists of trisodium citrate.

17. Pharmaceutical composition according to Claim 16, **characterized in that** the trisodium citrate concentration is between 1 and 15 mM, preferably between 7 and 10 mM.

18. Pharmaceutical composition according to any one of the preceding claims, **characterized in that** the pH is between 6 and 7.5, preferably between 6 and 7.

19. Pharmaceutical composition according to any one of the preceding claims, **characterized in that** it is free of sodium chloride and/or albumin.

20. Pharmaceutical composition according to any one of the preceding claims, **characterized in that** it has an osmolality of between 250 and 650 mOsm/kg, preferably between 300 and 550 mOsm/kg.

21. Liquid pharmaceutical composition according to any one of the preceding claims, **characterized in that** it comprises:
- 10-30 g/L of fibrinogen
- 10-300 mM of arginine
- 10-300 mM of glutamate
- optionally 1-15 mM of buffer, more particularly of trisodium citrate
- optionally 1-300 mM of another amino acid, more particularly 1-300 mM of proline and/or 1-300 mM of isoleucine and/or 1-300 mM of serine
- optionally 1-500 ppm of surfactant, preferably 20-400 ppm, more preferably 150-250 ppm
- optionally 5-1000 ppm of human albumin,
the pH of the composition being 6.0-8.0.

22. Liquid pharmaceutical composition according to any one of the preceding claims, **characterized in that** it comprises:
- 10-30 g/L of fibrinogen
- 20-200 mM of arginine
- 20-200 mM of glutamate
- optionally 7-10 mM of buffer, more particularly of trisodium citrate
- optionally 1-100 mM of another amino acid, more particularly 1-100 mM of proline and/or 1-100 mM of isoleucine and/or 1-100 mM of serine
- optionally 20-400 ppm of surfactant
- optionally 5-500 ppm of human albumin,
the pH of the composition being 6.0-8.0.

23. Liquid pharmaceutical composition according to any one of the preceding claims, **characterized in that** it comprises:
- 15-25 g/L of fibrinogen
- 150 mM of arginine
- 80 mM of glutamate
- 8.5 mM of trisodium citrate
- 200 ppm of surfactant, preferably of polysorbate80, of polysorbate20 or of Pluronic®F68
- optionally 1-100 mM of another amino acid, more particularly 1-100 mM of proline and/or 1-100 mM of isoleucine and/or 1-100 mM of serine
- optionally 5-1000 ppm of human albumin,
the pH of the composition being 6.0-7.0.

24. Pharmaceutical composition according to any one of the preceding claims, **characterized in that** the fibrinogen is obtained by blood plasma fractionation.
